# EUROPEAN PATENT SPECIFICATION

(11) **EP 3 908 832 B1**
(45) Date of publication and mention of the grant of the patent: **03.12.2025**
(21) Application number: 20739142.6
(22) Date of filing: 10.01.2020
(51) Int. Cl.: C07K 14/00, G01N 27/327, G01N 27/26, G01N 27/414, C12Q 1/6869, C07K 7/06, C07K 7/08, H10K 30/00, H10K 85/00, C07K 19/00

(54) **CONDUCTIVE SYNTHETIC PEPTIDES FOR MOLECULAR ELECTRONICS**
LEITFÄHIGE SYNTHETISCHE PEPTIDE FÜR MOLEKULARELEKTRONIK
PEPTIDES SYNTHÉTIQUES CONDUCTEURS POUR ÉLECTRONIQUE MOLÉCULAIRE

(30) Priority: 10.01.2019 US 201962790828 P
(43) Date of publication of application: 17.11.2021
(73) Proprietor: Roswell Biotechnologies Inc., San Diego, CA 92121 (US)
(72) Inventor: MERRIMAN, Barry, San Diego, California 92191 (US); GEISER, Tim, San Diego, California 92191 (US); ALAGARSWAMY GOVINDARAJ, Venkatesh, San Diego, California 92191 (US)
(74) Representative: Mewburn Ellis LLP
(86) International application number: PCT/US2020/013218
(87) International publication number: WO 2020/146823

(56) References cited:
- WO-A1-03/091458
- WO-A1-94/15628
- WO-A2-2012/139086
- US-A- 5 646 420
- US-A1- 2017 044 605
- US-A1- 2019 004 003
- US-B2- 9 139 614
- CHIU CHIN-YI ET AL: "Size-controlled synthesis of Pd nanocrystals using a specific multifunctional peptide", NANOSCALE, vol. 2, no. 6, 12 May 2010 (2010-05-12), United Kingdom, pages 927, XP093052876, ISSN: 2040-3364, DOI: 10.1039/c0nr00194e
- SOK YEE YEW ET AL: "Design of single peptides for self-assembled conduction channels", NANOTECHNOLOGY, INSTITUTE OF PHYSICS PUBLISHING, BRISTOL, GB, vol. 22, no. 21, 30 March 2011 (2011-03-30), pages 215606, XP020190344, ISSN: 0957-4484, DOI: 10.1088/0957-4484/22/21/215606
- ANONYMOUS: "GSP:BAD12258", 11 October 2012 (2012-10-11), XP093052882, Retrieved from the Internet <URL:http://ibis.internal.epo.org/exam/dbfetch.jsp?id=GSP:BAD12258> [retrieved on 20230608]

## Description

### FIELD

The present disclosure generally relates to synthetic peptides, and in particular to conducting synthetic peptides having alpha helical arrangements, which find use as molecular wires in molecular electronics.

### BACKGROUND

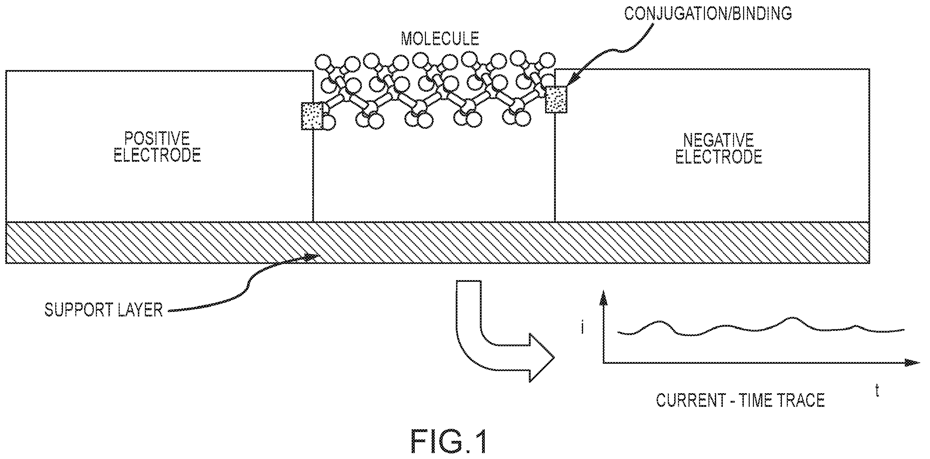

The broad field of molecular electronics was introduced in the 1970's by Aviram and Ratner. Their concept was to achieve the ultimate in scaling down of electrical circuits by using single molecules as circuit components. FIG. 1 illustrates their general concept of a molecule attached between nanoscale electrodes, as well as some conjugation group or mechanism that binds the molecule to the electrodes (small shaded squares). In some embodiments, a time-varying current, i, may pass through this molecule, as indicated in the inset plot of current versus time. In various embodiments, the molecular element may modulate this current, such as in logic processing circuits or sensing circuits.

Molecular circuit elements such as these could provide diverse functions, such as wires, resistors, switches, rectifiers, actuators or sensors, depending on the molecule and operating conditions. Of interest here is the application of such constructs as sensors, where molecular interactions with the molecule in the circuit provide a basis for single molecule sensing. Of interest as a circuit element are various forms of molecular wires, which can serve to provide a conducting connection between two points in the molecular circuit, as indicated in FIG. 1.

To those skilled in the art, the term molecular wire loosely refers to molecules of a regular or repetitive structure that have a relatively long, thin structure, and which are electrically conducting or semi-conducting. Examples of such molecular wires that have been well studied include carbon nanotubes, graphene ribbons, alpha helical proteins, double stranded DNA helices, and various synthetic organic polymers formed from aromatic rings, such as the well-known examples of polythiophenes (polymers formed of thiophene ring building blocks) and PEDOT (poly(3,4-ethylenedioxythiophene)) molecules.

In spite of the prior research into molecular wires, new molecules are still needed for use in electronic molecular circuits, and in particular, molecules having a precise molecular structure that can be produced by established methods of synthetic chemistry.

Chiu, Chin-Yi, Yujing Li, and Yu Huang. "Size-controlled synthesis of Pd nanocrystals using a specific multifunctional peptide." Nanoscale 2.6 (2010): 927-930 disclose a peptide-mediated synthesis of Pd NCs in aqueous solution with controllable size in the sub-10 nanometre regime. The specific multifunctional peptide Q7 selected using the phage display technique can bind to the Pd NC surface and act as a stabilizer to mediate Pd crystal nucleation and growth. At the nucleation stage, Q7 bound to and helped stabilize the different-sized small Pd NC nuclei achieved using different concentrations of the external reducing agent, NaBH4. At the growth stage, Q7 played the dual role of binding to and reducing the precursor onto the existing nuclei, which led to the further controllable growth of the Pd NCs. By using the variable sizes of nuclei as seeds, and by introducing different amounts of precursors Pd NCs with tunable sizes from 2.6 to 6.6 nm were achieved with good size distribution.

WO 94/15628 A1 discloses electrically conducting synthetic peptide complexes and methods for their preparation. The synthetic peptide complexes comprise first and second peptides which are spaced in a parallel relationship. The peptides possess a helical structure and are bound by a plurality of complexing agents. The complexing agents have redox potential asymmetry which provides the synthetic complex with an electrical bias. The synthetic peptide complexes are particularly useful in electronic devices, including molecular electronic devices.

### SUMMARY

In various embodiments of the present disclosure, electrically conducting synthetic peptides usable as molecular wires in molecular electronic circuits are described. The synthetic peptides herein can be produced by bottoms-up synthetic chemistry, such that the synthetic peptides can (a) comprise a precise molecular structure, e.g., to reduce variability in performance in molecular electronic circuits, (b) comprise specific attachment groups located at precise locations on the molecule as needed, and (c) be manufactured efficiently in large quantities at high purity and low cost.

In an aspect of the invention, disclosed herein is a synthetic peptide according to claims 1 to 6.

In various aspects, a terminus of a synthetic peptide, represented by the substructure [X¹X²]ₘX³, is customized for a particular utility, such as binding to a metal or conjugating to a biomolecule. In various embodiments, both instances of m cannot be zero, in which case one terminus of the synthetic peptide retains a [X¹X²]ₘ substructure.

In a further aspect of the invention, disclosed herein is a molecular electronics circuit according to claim 7.

In a further aspect of the invention, disclosed herein is a sensor according to claim 8.

In various embodiments, a CMOS chip device comprises an array of these sensors.

In a further aspect of the invention, disclosed herein is a method of sequencing a DNA molecule according to claim 9.

In a further aspect of the invention, disclosed herein is a molecular electronics circuit according to claim 10.

In a further aspect of the invention, disclosed herein is a sensor according to claim 11.

In various embodiments, a CMOS chip device comprises an array of these sensors.

In a further aspect of the invention, disclosed herein is a method of sequencing a DNA molecule according to claim 12.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the detailed description and claims when considered in connection with the drawing figures:
FIG. 1 illustrates the general concept of a molecular electronic circuit, with a conducting molecule completing a circuit between nanoelectrodes;
FIG. 2 illustrates the general concept of engaging a binding probe molecule onto a molecular wire in an electronic circuit, to act as a sensor capable of detecting a binding event between the probe binding and its target, in accordance with various embodiments;
FIG. 3 illustrates a binding probe comprising a molecular wire wired directly into the current path, using two molecular wire elements or arm molecules, in accordance with various embodiments;
FIG. 4 illustrates a binding probe wired directly into the current path, with the connection made via molecular wires connecting to an internal protein alpha-helix structure within the probe, in accordance with various embodiments;
FIG. 5 illustrates a binding probe wired directly into the current path, with the connection made via molecular wires connecting to an internal protein beta-sheet structure within the probe, in accordance with various embodiments;
FIG. 6 illustrates a binding probe wired directly into the current path, with additional molecular wire connections made to stabilize or orient the probe, or increase the conductivity of the connection to the probe, in accordance with various embodiments;
FIG. 7 illustrates a schematic of a binding probe directly wired directly into a circuit using two molecular wire connectors, as well as also having a one-point conjugation to a molecular wire, and utilizing one pair of electrodes to measure the combined conduction, in accordance with various embodiments;
FIG. 8 illustrates a schematic of a binding probe directly wired directly into a circuit using two molecular wire connectors, as well as also having a one-point conjugation to a molecular wire, and utilizing two pairs of electrodes to measure these two modes of conduction independently, in accordance with various embodiments;
FIG. 9 illustrates a schematic where the binding probe is an IgG antibody, shown with one Fab domain bound to its antigen binding target, directly wired into the current path of a circuit, wherein specific internal beta-sheets are used for the contacts, and molecular wire arms provide conducting connections to the electrodes, in accordance with various embodiments;
FIG. 10 illustrates a schematic of a nucleic acid hybridization binding probe molecule, composed of single stranded RNA or DNA or other XNA nucleic acid analog oligomer, directly wired into the current path of a circuit using two molecular wires, in accordance with various embodiments, wherein sites at or near the termini of the single strand are conjugated to molecular wire connectors to provide a conducting connection to the electrodes;
FIG. 11 shows a protein structure view of the *E. coli* Pol I Klenow Fragment polymerase enzyme, illustrating the presence of alpha-helix, beta-sheet, and connecting loop structures;
FIG. 12 shows a schematic of polymerase enzyme molecule conjugated onto a molecular wire in an electronic circuit, to act as a sensor capable of detecting the activity of the enzyme, in accordance with various embodiments;
FIG. 13 shows a schematic of polymerase enzyme directly wired into the current path of a circuit, to act as a sensor capable of detecting the activity of the enzyme, in accordance with various embodiments, wherein a specific internal alpha-helix is used for the contact points, and molecular wire arms provide conducting connections from these contacts to the electrodes;
FIG. 14 shows a schematic of polymerase enzyme directly wired into the current path of a circuit, using two molecular wire connectors, and where additional molecular wire connecting arms are wired to provide stabilization and fixed spatial orientation, as well as additional conducting connections to the electrodes or substrate;
FIGS. 15A-E show various representations of SEQ ID NO: 4: FIG. 15A a ribbon diagram, FIG. 15B a ball and stick model of the sequence backbone, FIG. 15C a space filling model, FIG. 15D a ball and stick model of the complete molecule, and FIG. 15E a space filling model.
FIGS. 16A-C show molecular renderings of SEQ ID NO: 4, with dashed lines representing hydrogen bonds in the helix and salt-bridges between the amino acid side-chains;
FIGS. 17A-C show molecular renderings of a peptide alpha-helix based on repeating EEEERRRR (SEQ ID NO: 2) (E4R4) alpha helix motif, with dashed lines representing hydrogen bonds in the helix and salt-bridges between the amino acid side-chains;
FIG. 18 shows molecular renderings of a peptide alpha-helix based on repeating EAYAR (SEQ ID NO: 3) alpha helix motif, with dashed lines representing hydrogen bonds in the helix and salt-bridges between the amino acid side-chains;
FIG. 19 shows the crystal structure for the specific major Pilin chain, Type IVa, from this bacterium, (Protein Data Base entry PDB ID 2M7G_A). The X-ray crystal structure, visualized with a ribbon plot and superimposed ball-and-stick model showing all side chains, shows that the pilin protein has an alpha helical structure, 13 turns long, and three aromatic ring side chains of the are visible;
FIG. 20 shows current distributions observed for 15nm long synthetic peptides SEQ ID NO: 15 and SEQ ID NO: 19, based on EAAAR (SEQ ID NO: 1) and EEEERRRR (SEQ ID NO: 2) repeating alpha helix motifs, respectively, showing a higher conductivity for synthetic peptide SEQ ID NO: 15; and
FIG. 21 shows a current vs. time trace of an experiment in which a 25nm reference synthetic peptide having SEQ ID NO: 21 and based on the EAAAR (SEQ ID NO: 1) repeating alpha helix motif is conjugated to a DNA oligonucleotide at a central cysteine and used to sense polymerase binding to the primed template and nucleotide binding into the polymerase pocket.

### DETAILED DESCRIPTION

In various embodiments of the present disclosure, a synthetic peptide is described. In various embodiments, a synthetic peptide herein exhibits electrical conductivity and finds use in molecular electronics, such as single-molecule electronic molecular sensors, and in electrical circuits integrated into CMOS semiconductor chip devices.

The synthetic peptide comprises an overall alpha helix conformation, or comprises at least one internal alpha helical segment.

The synthetic peptide has an amino acid sequence designed to provide an overall alpha helix conformation to the peptide, or has an amino acid sequence designed to provide at least one internal alpha helical segment. The synthetic peptide herein comprises repeating helical segments or "helical motifs."

In various embodiments, a synthetic peptide herein comprises a linear primary structure and, accordingly, two opposite ends to the sequence that may be referred to as termini, a first terminus at or near the N-terminus of the peptide and a second terminus at or near the C-terminus of the peptide. Either or both termini may be used for site specific conjugation, such as attachment to metal electrodes or attachment to external chemical groups.

The synthetic peptide herein also comprises an internal site between the termini and along the peptide amino acid sequence that can be used to conjugate another molecule to the synthetic peptide in a site specific, selective manner. The internal site may comprise a single cysteine amino acid (abbreviated C or Cys), or a lysine (K or Lys), or a tyrosine (Y or Tyr), or an amino acid with a chemical modification that is used for conjugation, such as the addition of a thiol, a biotin, an azide, or a click chemistry group.

### Definitions

As used herein, the term "peptide" refers to any contiguous single chain of amino acids, wherein the amino acids are standard, non-standard or modified, or amino-acid analogs that engage in a peptide bond. In various embodiments, peptides herein may be in the range of 10 to 300 amino acids long, or 20 to 200 amino acids long.

As used herein, the term "motif" refers to a feature within a synthetic peptide in accordance with the present disclosure. In various examples, a motif may be an alpha helical motif, meaning a segment of amino acids within a peptide having alpha helical secondary structure. In other examples, a motif may be one or more amino acids within a peptide having another functional purpose, such as a motif comprising a material binding peptide usable to anchor a synthetic peptide to a metal. In other aspects, a motif may comprise a chemical substituent, such as a functional group in organic chemistry (*e*.*g*., amine, thiol, etc.), or a motif may comprise a chemical linker, (*e.g.,* one or two amino acids, a string of a three amino acids, a (poly)ethoxylate tether, a 1,4-phenylene linkage, etc.).

As used herein, the term "alpha helix" refers to the helical secondary structure of a peptide, as used in the context of describing protein structural elements in the field of X-ray crystallography. Herein, a segment of a synthetic peptide may have alpha helical secondary structure, and the segment may alternatively be referred to as a "helical segment" or a "helical motif." A synthetic peptide herein may comprise one or more helical motifs, such as repeating alpha helical motifs. In some instances, the term "alpha helix" may be used in place of the term "synthetic peptide" for peptides substantially in an alpha helical secondary conformation when ignoring binding peptides at the two ends of the peptide. In various examples, "an alpha helix" may refer to a synthetic peptide usable as a conducting bridge molecule in a molecular electronics circuit.

As used herein, the term "molecule" refers to a covalently bonded assembly of atoms, or a similarly well-defined and stably bound assemblage of atoms.

As used herein, the term "molecular electronics" refers to electrical circuits which incorporate a single molecule or small molecular complex involving a few molecules, as an element in an electrical circuit. Such small complexes may involve just two or three molecules, and in various embodiments less than 10, or less than 30. A molecular electronics sensor is an example of molecular electronics.

As used herein, the term "molecular wire" refers to a relatively long and thin molecule or assemblage of a small number of molecules, which can conduct electricity when incorporated into a circuit, such as by spanning a pair of electrodes or contact points for charge transfer. Such assemblages may be conductors or semi-conductors and may have linear or nonlinear current versus voltage characteristics. They may also exhibit a bandgap, which suppresses conduction below a threshold voltage. The length of a molecular wire may be on the order of several nanometers up to several microns. A synthetic peptide herein, capable of electrical conductivity, may function on its own as a molecular wire in a molecular electronics circuit. Or synthetic peptides in accordance with the present disclosure may be assembled into molecular wires comprising larger structures, such as, *e*.*g*., comprising bundles of peptides arranged like filaments in a yarn. In cases where a synthetic peptide can be used directly in a molecular electronic circuit, the terms "synthetic peptide" and "molecular wire" may be used interchangeably.

As used herein, the term "synthetic peptide" refers to a peptide that is manufactured rather than naturally occurring, *i*.*e*., a peptide that has been designed, engineered and/or produced by artificial means. As such, "synthetic" should not be interpreted narrowly as meaning a peptide assembled entirely by a linear or convergent chemical synthesis based on organic chemistry methods. A synthetic peptide herein may also be made by protein expression and genetic engineering. That is, a synthetic peptide may be prepared in a protein expression system, expressing a synthetic DNA gene inserted into such a system. Further, a synthetic peptide produced by protein expression genetic engineering may be further functionalized by organic synthesis, such as to add a functional group, remove a protecting group, and so forth. A synthetic peptide herein, although manufactured, may be inspired by naturally occurring peptides, and may comprise naturally occurring amino acid sequences.

As used herein, the term "terminus," or plural, "termini," refer to the ends of an amino acid sequence of a synthetic peptide in accordance with the present disclosure, and allow for a distinction between an alpha helical and conductive core of a synthetic peptide and the end regions that provide other functionality such as conjugation for binding to electrodes or for tagging. The "ends" of the sequence should not be interpreted as the actual physical end of the sequence (an atom, or one amino acid), but should be interpreted more broadly as the ending region of a synthetic peptide, which may be sterically longer than just one amino acid. In other words, a terminus to a synthetic peptide, either the N- or C- end, may comprise a single amino acid, a sequence of amino acids, various combinations of linkers and functional groups, various combinations of linkers and short sequences of amino acids, and so forth. In various embodiments, a terminus to a synthetic peptide may comprise a cysteine at the end of the sequence, so that a thiol (-SH) functional group is available for conjugation to a metal. In other examples, a terminus to a synthetic peptide may comprise a single amino acid that has been functionalized to include an unnatural appendage, such as an azide group. In other examples, a terminus to a sequence may comprise a material binding peptide, or a double, triple, or quadruple repeat of a material binding peptide having specificity to a particular metal such as gold or palladium. In other examples, a terminus of a synthetic peptide may comprise metal binding groups such as sulfide -S- atoms strung together with intervening linkers comprising one, two or three amino acids.

As used herein, the terms "sequence identity" and "percent sequence identity," in the context of two or more peptide sequences, refer to two or more sequences or subsequences that have a specified percentage of amino acid residues that are the same, when compared and aligned for maximum correspondence, as measured using one of the sequence comparison algorithms for such amino acid sequence comparisons (*e*.*g*., BLASTp or other algorithms available to persons of ordinary skill in the art) or by visual inspection. Depending on the application, the percent sequence identity can exist over a region of the sequence being compared, *e*.*g*., over an alpha helical segment, or, alternatively, exist over the full length of the two sequences to be compared. For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

As used herein, the term "polymer" refers to a molecule that is a chain of chemical building blocks, taken from definite finite set of building block molecules, and linked to each other through a covalent bond.

As used herein, the term "aromatic ring" takes on the common meaning in organic chemistry of a ring of sp2 hybridized carbon atoms, with or without intervening heteroatoms having a pair of electrons, wherein the electrons in the orbitals of each of the atoms in the ring are delocalized. The term aromatic molecule or aromatic amino acid refers to such an entity that comprises an aromatic ring in its molecular structure.

It is a convention used herein that all amino acid or protein sequences are written from the N-terminus to the C-terminus.

As used herein, the term "binding probe" refers to a molecule or molecular complex that preferentially binds a specific target molecule or family of target molecules. An antibody or antibody fragment is an example of a binding probe. An enzyme, such as a polymerase, is also an example of a binding probe. In various embodiments herein, a binding probe may be attached to a synthetic peptide used as a bridging molecular wire between electrodes in a molecular electronics circuit.

As used herein, the term "enzyme" refers to a molecule or molecular complex, typically comprising one or more proteins, that catalyzes a chemical reaction. The term polymerase refers to such an enzyme that synthesizes a DNA or RNA complement to a DNA or RNA single stranded template. In various embodiments, an enzyme acts as a binding probe in a molecular electronics circuit.

As used herein, the term "salt bridge" refers to a weak bonding phenomena that forms between positively and negatively charged residues of amino acids present in a peptide located proximate in space so that such a bond can form.

As used herein, the term "bridge" or "bridge molecule" or bridging, refers to molecules that may be used to form conducting bridges between electrodes, which apply to all the synthetic peptides discussed herein having suitable attachment groups at the termini. A related term is "arm" or "arm molecule," which refers to the use of synthetic peptides in a modality that span between an electrode and a biomolecule or probe molecule, in contrast to spanning between electrodes, and would also apply to all the synthetic peptides discussed herein having suitable attachment groups at the termini.

As used herein, the term "electrode" refers to one of the electrical contact points in an electrical circuit, that serves to conduct electrons through the completed circuit. Such electrodes are typically made of metal or doped semiconductors, are relatively highly conductive, and may further have their surfaces derivatized to promote proper electrical and mechanical connection, such as with molecular wires.

### Synthetic peptides having structural motifs to promote use as conducting molecular wires in molecular electronic circuits

In various embodiments, a synthetic peptide herein comprises specific structural motifs to enhance electrical conductivity through the peptide structure and to provide various conjugation sites such as at one or both termini of the peptide and between the termini.

The synthetic peptide in accordance with the present disclosure is represented by the following generalized structure: [X¹X²]ₘX³[X⁴]ₙX³[X²X¹]ₘ as defined in the claims, wherein [X⁴]ₙ represents a repeating alpha helical motif X⁴, repeated n times, and wherein the combination of [X¹X²]ₘX³ at both ends of the sequence represent the termini of the synthetic peptide. There are two important aspects to the generalized structure. First, the motifs X¹, X², X³, and X⁴ in the generalized structure are not limited to single amino acids, but instead can be amino acid sequences. As an example, X⁴ can be EAAAR (SEQ ID NO: 1), which is not a single amino acid. Secondly, it is important to understand that *each* instance of a motif within a repeated substructure, such as X¹, X² and X⁴ appearing in brackets in the generalized structure, are selected independently. For example, in the substructure [X⁴]ₙ when n=3, *i.e.,* [X⁴]₃, which may be represented as X^{4'}X^{4"}X^{4‴}, X^{4'}, X^{4"} and X^{4‴} may be different motifs, *e*.*g*., different amino acid sequences. At least one instance of X⁴ comprises a conjugation site. Also, each of the two X³ motifs on either end of the peptide need not be identical, nor do each of the termini [X¹X²]ₘX³ of a synthetic peptide need to be identical. In other words, the generalized structure above may suggest a certain symmetry through the midpoint of a synthetic peptide fitting the formula, but any species encompassed by the general structure need not have such symmetry. For example, an N-terminus represented by [X¹X²]ₘX³ may be designed to bind to gold, whereas the other terminus, the C-terminus represented by [X¹X²]ₘX³ may be designed to bind to palladium, such that the synthetic peptide species can bridge between electrodes of different metals. In various embodiments, one instance of m may be 0 while the other instance of m may be 1, 2, or 3. That is, in various embodiments, m is not 0 for both termini portions of the synthetic peptide. In various examples, one terminus [X¹X²]ₘX³ may include a tag sequence. In various embodiments, one instance of m is 0, while the other instance of m is not zero, such that one terminus of the synthetic peptide ends in X³, which can, for example, be designed for click chemistry conjugation to a biomolecule, whereas at the other terminus, the substructure [X¹X²]ₘ, wherein m = 1, 2 or 3, can be designed to bind to a metal.

Lastly, as a general rule, the general structure above should not imply relative lengths of the motifs in the structure, *i.e.,* the segments of a synthetic peptide. In other words, the general structure as it's written might suggest that the internal helical core portion represented by [X⁴]ₙ is shorter in length than the combination of the two termini represented by [X¹X²]ₘX³, which is not necessarily the case. For example, n may be 21 and X⁴ may be a peptide of 5 amino acids, such that [X⁴]ₙ is 105 amino acids in length.

The nature and scope of the motifs X¹, X², X³, and X⁴, and the range of the repeat units m and n, are defined herein below and in the claims.

In various embodiments, incorporation of specific structural motifs, particularly helical motifs, promote alpha helical structure to at least a portion of the synthetic peptide. Depending on the amino acid sequence within these alpha helical segments, electrical conductivity is promoted through the synthetic peptide. These motifs may, for example, comprise:
(1) amino acids with side chains of opposing charge that may form salt-brides between the residues in the alpha-helical conformation, at i and i+5 positions in the peptide chain, or at i and i+4 positions in the peptide chain;
(2) amino acids with aromatic ring residues;
(3) amino acids with aromatic rings residues at i and i+4 or i and i+5 positions, so that such rings may be spatially neighboring in the alpha-helical conformation;
(4) hydrocarbon chain staples to stabilize an alpha-helical structure, where such staples may be between i and i+4 or i+5 residues in the chain, to staple adjacent sites in the alpha-helical conformation; or
(5) amino acid sequence motifs mimicking or inspired by biological conducting proteins, which have a biological function of charge transfer in nature.

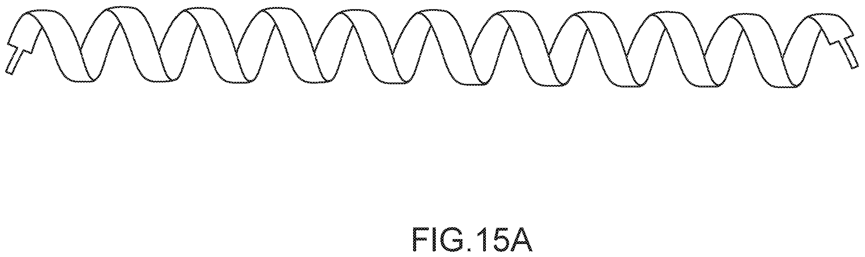

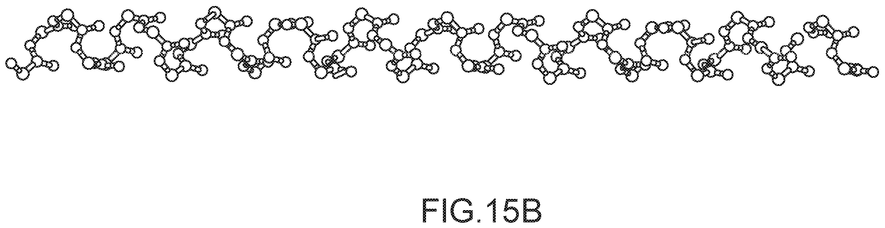

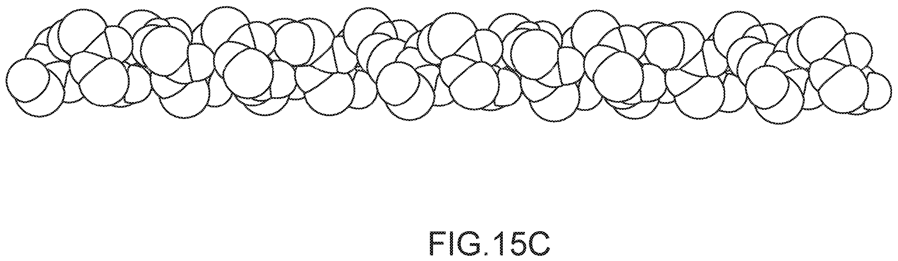

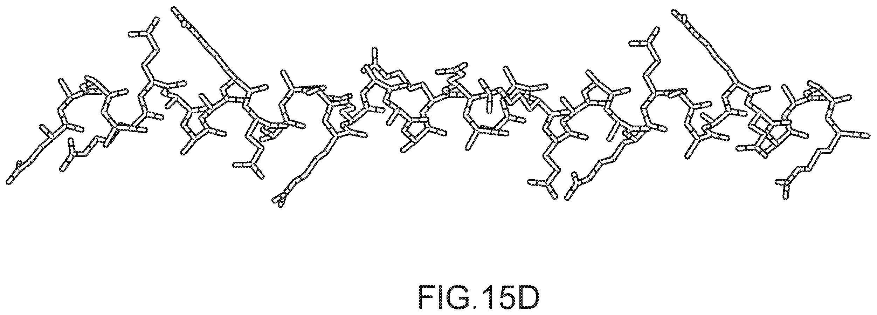

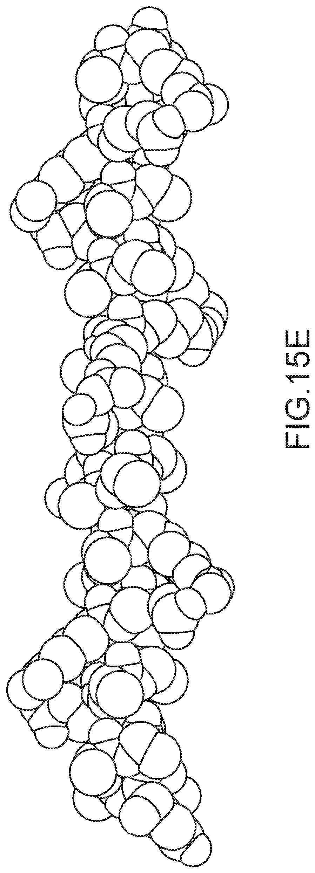

For general illustration of the alpha helix peptide concept, FIG. 15 shows five different graphical representations of an alpha-helical peptide. The peptide in this example consists of 40 amino acids, with a primary amino acid sequence (written using standard AUG amino acid code single letter designations) consisting of 8 repeats of the motif EAAAR (SEQ ID NO: 1) (E= Glutamic Acid, A= Alanine, R = Arginine), or in compact notation as 8x(EAAAR) (SEQ ID NO: 4), or, written explicitly, in standard direction from N-terminus to C-terminus, EAAAREAAAREAAAREAAAREAAAREAAAREAAAREAAAR (SEQ ID NO: 4).

FIGS. 15A-E show various representations of SEQ ID NO: 4. FIG. 15A is a protein ribbon diagram of the alpha helical structure of the peptide, FIG 15B is a ball-and-stick model of the protein peptide bond backbone, FIG. 15C is a space-filling model of the protein peptide bond backbone, FIG. 15D is a ball-and-stick model of the complete molecular structure, including the amino acid side-chains, and FIG. 15E is a space-filling model of the complete molecular structure, including the amino acid side-chains.

The general structure of an alpha helix consists of 3.6 amino acids per helical turn, a length of 0.54 nm per helical turn, and a helical core diameter of 1.2 nm. Thus, the example peptide of SEQ ID NO: 4 shown in FIGS. 15A-E has a length of 40 amino acids, or 11.1 helical turns, or 6.0 nm.

In various embodiments, a synthetic peptide in accordance with the present disclosure comprises a sequence having at least 80%, at least 85%, at least 90%, at least 95%, or at least 98% sequence identity to SEQ ID NO: 4. In various embodiments, the synthetic peptide comprises SEQ ID NO: 4. In various embodiment, such a synthetic peptide functions as a conductive molecular wire for a molecular electronics circuit. In various embodiments, this alpha helical structure may be the central core of a larger synthetic peptide.

In various embodiments of the present disclosure, the alpha-helical peptides may be designed to have a precise length in the range of 3 nm to 100 nm, or in some embodiments, 5 nm to 50 nm, or in other embodiments 7 nm to 30 nm, 10 nm to 25 nm, or 10 nm to 15 nm.

The synthetic peptides may be made by established methods of peptide synthesis, such as those known to one skilled in the art of synthetic organic chemistry and biochemistry. These methods may include, but are not limited to, solid phase synthesis or solution phase synthesis of peptide-bonded amino acids and may further include use of chemical ligation of such synthetic fragments to efficiently produce longer peptides. The amino acid building blocks used may comprise the standard 22 proteinogenic amino acids that appear in biology, as well as the use of non-proteinogenic amino acids, including the so-called Non-Standard Amino Acids (NSAA) or Unnatural Amino Acids (UAA), such as the well-known examples of chemically modified forms of Phenylalanine (F), designated as pAcF (pacetyl-F), pAzF (pazido-F) and pBpF (pbenzoyl-dl-F).

The synthetic peptides in accordance with the present disclosure may also be made by expression of a synthetic gene, representing a desired peptide sequence. Such protein expression methods are well known, and typically involve cloning a synthetic DNA segment ("gene") into a bacteria or other expression vector, expressing and purifying out the resulting protein of interest. For such expression systems, a capture tag peptide (such as His tag or FLAG tag or others known in the art) may be added at one termini, C- or N-, of the target peptide of interest, and this tag may or may not be removed post capture, in various embodiments. If such a small tag or related peptide remnant is left in place, it generally will not impact the utility of the synthetic peptides as described herein.

In the case of expression, it is also possible to add NSAAs into the peptide, if expression systems that are made for such purposes are used, as in the case of so-called "expanded genetic codes." Such expression systems, which rely on the use of a bacterial vector with a modified genetic code, co-expression of customized transfer-RNAs charged with the desired NSAA, are well known to those skilled in the art of expanded genetic codes, for example, such systems for expression of proteins with NSAAs have been pioneered by biologists Peter G. Schultz and George M. Church. Such systems, based on *E. coli,* have been used to express proteins that include over 70 different NSAAs to date.

Binding groups at or near the termini of the peptide, which may be positioned at or near the ends of an alpha-helical segment, which are useful for conjugation of a peptide into molecular circuits, may comprise the amino acid cysteine (C), which is useful for thiol-based conjugation, such as thiol-metal binding to metallic electrode surfaces, or which is also useful for cysteine-maleimide selective binding to maleimide as a conjugation chemistry, or for cysteine-cysteine or cysteine-sulfur sulfide bridge-based conjugation. The binding groups also may comprise a cysteine rich motif, CC, CCC, CCCC (SEQ ID NO: 32), CCCCC (SEQ ID NO: 33), or the "FLASH" tetra-C motif CCXXCC (X = any amino acid), such as CCCGCC (SEQ ID NO: 5) or CCPGCC (SEQ ID NO: 35). The conjugation groups at or near the termini of a helical segment may also include amino acids that have groups capable of binding to cognate-derivatized surfaces, such as azide or amine groups, or groups capable of participating in click chemistry binding. The conjugation groups at or near the termini of a helical segment may also comprise Material Binding Peptides. Material Binding Peptides are peptides in the range of 5 to 15 amino acids long having an amino acid sequence capable of binding to specific materials. Many such Material Binding Peptides are known to those skilled in bioconjugation. One example, the Gold Binding Peptide of Brown, known to selectively bind to gold, has the amino acid sequence MHGKTQATSGTIQS (SEQ ID NO: 6). See, for example, S. Brown, "Metal-recognition by repeating polypeptides," *Nature Biotechnology,* 15, 269-272, 1997. Other known metal binding peptides include, but are not limited to, WAGAKRLVLRRE (SEQ ID NO: 7), VSGSSPDS (SEQ ID NO: 8), TGTSVLIATPYV (SEQ ID NO: 9), LKAHLPPSRLPS (SEQ ID NO: 10) and QQSWPIS (SEQ ID NO: 16). The last example is a palladium binding peptide.

Furthermore, binding groups may comprise such binding peptides arranged as a series of tandem repeats, separated by short spacer peptides. For example, there may be three copies of the gold binding peptide, separated by the peptide linker -GSG-, such as the amino acid sequence MHGKTQATSGTIQS-GSG-MHGKTQATSGTIQS-GSG-MHGKTQATSGTIQS (SEQ ID NO: 11). Such a group may be included at either terminus of the peptide molecular wire, and may comprise an additional short peptide linker, such as a -GSG- or other short peptide composed of G and S, or other common flexible, water soluble peptide linker sequences, to offset it from the primary alpha-helical segment, so as not to disrupt the helical conformation. In various embodiments, there may be 2 to 5 such repeats in the binding group.

In various embodiments, a synthetic peptide in accordance with the present disclosure comprises a sequence having at least 80%, at least 85%, at least 90%, at least 95%, or at least 98% sequence identity to SEQ ID NO: 11. In various embodiments, the synthetic peptide comprises SEQ ID NO: 11. In various embodiment, such a synthetic peptide functions as a conductive molecular wire for a molecular electronics circuit. In various embodiments, this sequence may be part of one or both termini of a larger synthetic peptide.

In various embodiments, an internal binding site in the peptide, such as near a central location along the amino acid sequence, may comprise a cysteine amino acid at a specific site to provide for cysteine based conjugation, such as cysteine-maleimide conjugation. The internal binding site may also comprise an amino acid, standard, nonstandard, or modified, at a specific position in the peptide with a residue, such as an azide or an amine group, which can undergo a specific conjugation reaction. For example, an amino acid comprising an azide functionality can be used for click chemistry conjugation reactions. In various embodiments, an internal binding site may be included in an alpha helical motif, such as in a synthetic peptide comprising an alpha helical core segment further comprising repeating alpha helical motifs where one or more of the alpha helical repeats includes a cysteine.

One or more isolated amino acids capable of functioning as an internal binding site in the peptide will typically not disrupt the alpha helical structure of the peptide, even if they depart from a precise repeated motif that defines the helical structure, or if placed between two such tandem repeats of the motif. In some embodiments, the location of the internal binding site in the peptide chain, and the end groups of the peptide, will be spaced such that in the helical conformation, the peptide will be bound in place in a circuit and conjugated with the ancillary molecules without distortion to the helical geometry, while allowing the ancillary molecules to be oriented as desired relative to the electrodes and substrate of the circuit. For example, the peptide end groups, when bound to the electrodes in a minimal energy configuration, and with the helical segment in its standard minimal energy conformation, can result in the internal binding group being oriented away from the underlying substrate of the circuit, maximally available in solution for the other ancillary molecules to bind.

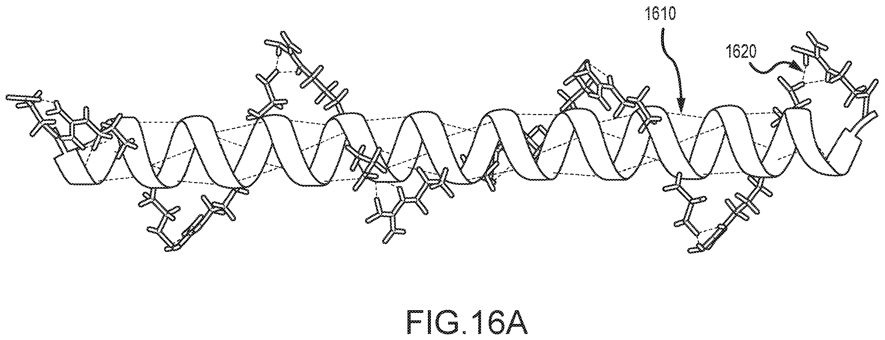

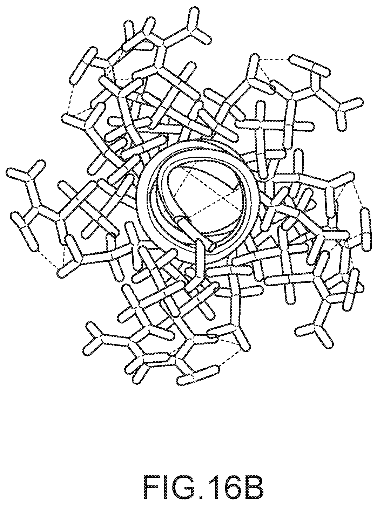

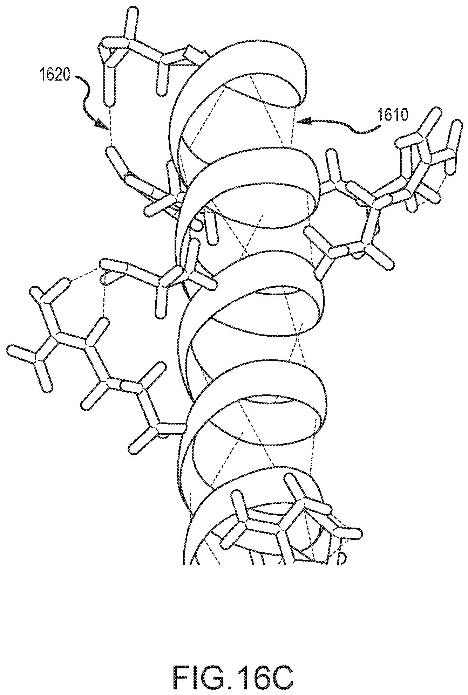

The use of salt bridges to both stabilize the helical structure and provide conduction paths is illustrated by the examples in FIGS. 16, 17 and 18. The salt bridges are formed between amino acids that are spatially nearby along the 3-D alpha helical structure. These typically are at i and i+4 or i+5 positions in the amino acid sequence. These examples illustrate the principles but do not restrict the scope of the present disclosure, which encompasses other amino acid sequences that would achieve similar effects. In general, the salt bridging can occur between the residues of positive and negatively charged amino acids, such as the standard amino acids R, H, K (positive) and E, D (negative). In various embodiments, charged NSAA could be used as well.

FIGS. 16A-C show a molecular rendering of SEQ ID NO: 4, which is based on repeating EAAAR (SEQ ID NO: 1) helical peptide motif, with dashed lines 1610 in the rendering representing the hydrogen bonds in the helix and dashed lines 1620 representing the salt-bridges between the E and R amino acid side chains (other side chains not shown for clarity). The intramolecular hydrogen bonds and salt bridges provide favorable conduction paths for electrons traversing the molecular wire comprising repeats of the motif EAAAR (SEQ ID NO: 1), when the molecular wire is connected in a molecular circuit. The salt bridges, shown as interacting side chains of i and i+4 amino acids E and R, further help to stabilize the helical structure.

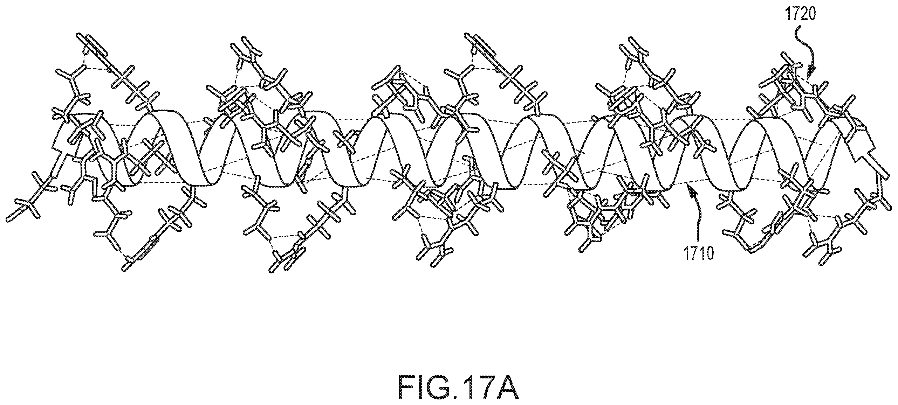

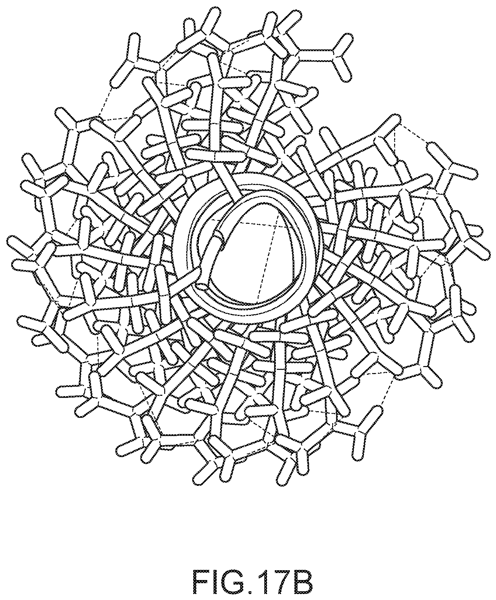

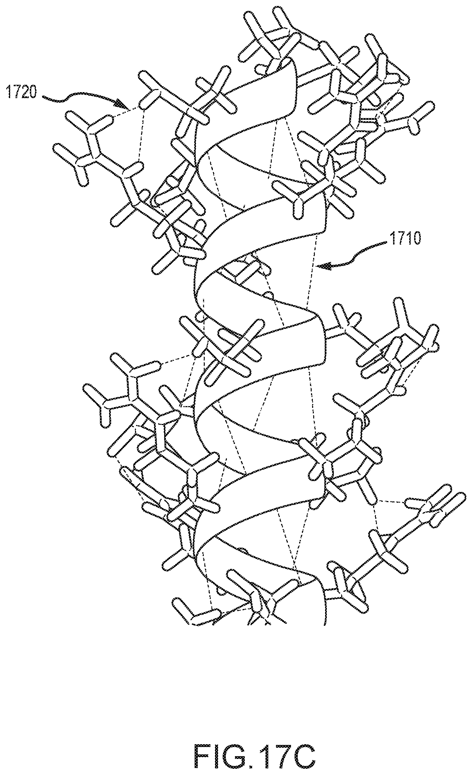

FIGS. 17A-C show molecular renderings of a synthetic peptide comprising repeats of the EEEERRRR (SEQ ID NO: 2) (E4R4) helical peptide motif, with dashed lines 1710 in the rendering representing the hydrogen bonds in the helix and with dashed lines 1720 in the rendering representing the salt-bridges between the E and R amino acid side-chains (other side chains not shown for clarity).

In various embodiments, enhanced conductivity is achieved by incorporating amino acids comprising aromatic rings in the synthetic peptide. Aromatic rings are electron rich and provide support for electron conduction through proteins. By incorporating such aromatic ring side chains into the alpha helix segment of a synthetic peptide, conduction through the peptide may be further enhanced.

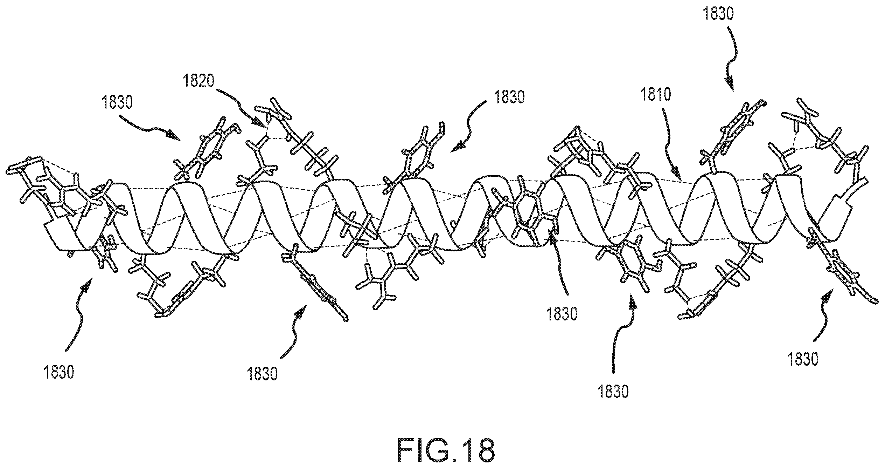

FIG. 18 shows such a synthetic peptide, based on repeats of the motif EAYAR (SEQ ID NO: 3), with dashed lines 1810 indicating the hydrogen bonds, and where the side chains of E, R and aromatic Y (1830) are shown. The E and R side chains provide salt bridges 1820, and the Y groups (tyrosine) 1830 provide aromatic rings to enhance conductivity. The naturally occurring amino acids with aromatic rings are F, W, Y, P (phenylalanine, tryptophan, tyrosine, proline), and these as well as non-sulfonated aromatic amines (NSAA) may be used for this effect. In various embodiments, the aromatic amino acids are spaced by i and i+4 and or i and i+5 in the peptide chain, so that the aromatic rings are spatially near each other in the helical structure, to provide a continuous conduction path for electrons hopping from ring to ring.

In various embodiments, enhanced conductivity is achieved by including synthetic peptide segments that are highly similar to peptide segments of biological conducting helical proteins, such as proteins that are known to carry out electron transfer or charge transfer in various biological processes, or that are part of complexes that carry out such conducting functions.

An example of such a family of biological proteins are bacterial Pilins, such as, for example, the Pili proteins of *Geobacter sulfurreducens,* a gram-negative metal-reducing proteobacterium. This genus was discovered in 1987 and has been well studied for its metal-reducing and electron conducting properties. See, for example, K. Xiao, et al., "Low Energy Atomic Models Suggesting a Pilus Structure that could Account for Electrical Conductivity of *Geobacter sulfurreducens* Pili," *Scientific Reports,* March 22, 2016, (DOI: 10.1038/srep23385).

In the biological system, the individual Pilin protein chains are twisted into a helical superstructure "filament" formed from 21 chains, which plays a key role in electron transport in the bacteria.

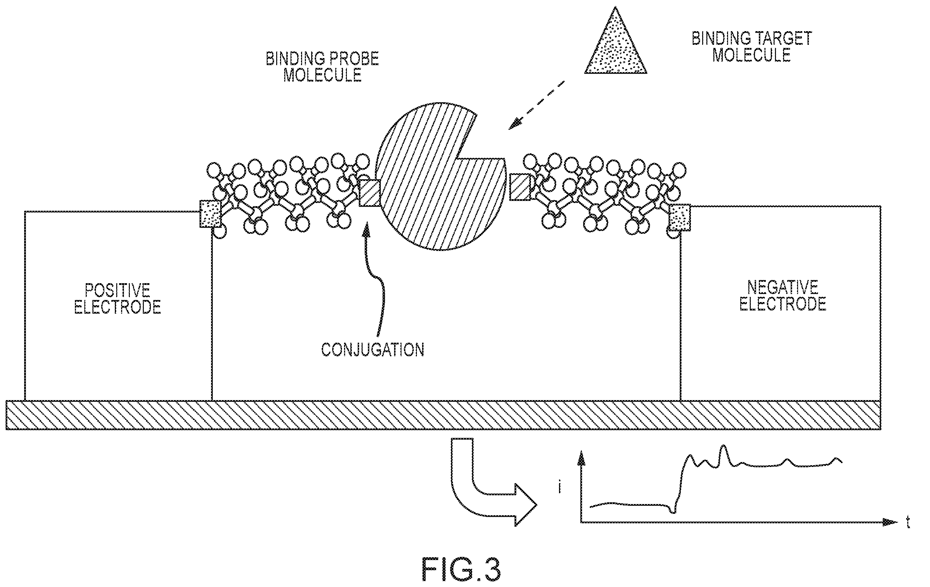

Xiao, et al., *supra,* illustrates the basis of the conducting properties of the biological Pilin protein and Pilin filaments. Figure 3 in Xiao, et al., *supra,* shows results for a predicted model structure of the *G*. *sulfurreducens* pilus: (A) The complete filament superstructure model containing 21 single-chain helical protein subunits, each with a different shading; (B) shows the geometric parameters of the superstructure; and (C) shows an end view of the superstructure.

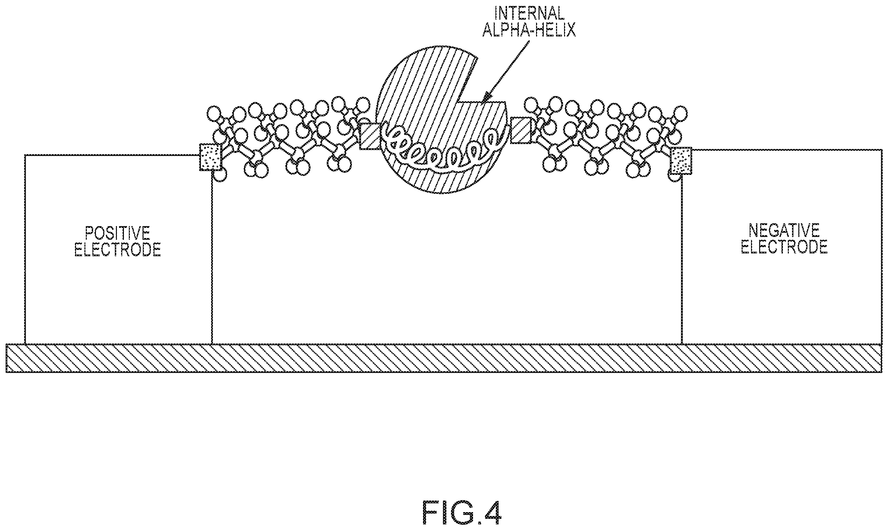

Figure 4 in Xiao, et al., *supra,* shows how the aromatic rings are organized through the filament superstructure, with a chain of spatially adjacent aromatic rings forming a continuous conduction path through the superstructure (A, B, C and D) and the comparison of this aromatic ring structure to a *Geobacter* homology model from *Pseudomonas* (E, F): (A) Shows the aromatic rings in the pilus model; (B) shows details of the neighboring aromatic rings, which repeat through the length of the filament. The closest atoms between rings are 3.5 Å apart. The centers of the rings are 4-5 Å apart. "P" denotes a reference protomer. "P + 3" and "P + 4" denote 3rd and 4th protomers, respectively, farther along the helical assembly; (C) End view of the model containing 21 monomers (model length 275 Å); (D) Cross section of the model filament (22 Å thick), in which residues from different monomers are shown in different shadings. Aromatic rings of Phe1, Phe24 and Tyr27 are opaque and off-center in the cross section; (E) Cross section of the *Geobacter* homology model based on Pseudomonas; (F) Absence of a central channel in the model (left) vs. the central channel in the homology model (right).

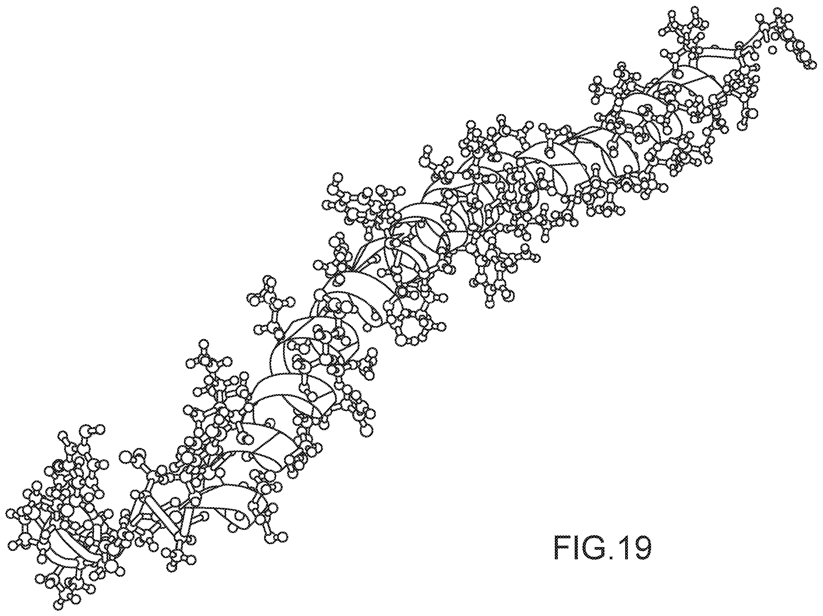

FIG. 19 herein shows the crystal structure for the specific major Pilin chain, Type IVa, from this bacterium, (Protein Data Base entry PDB ID 2M7G_A). The X-ray crystal structure, visualized with a ribbon plot and superimposed ball-and-stick model showing all side chains, shows that the pilin protein has an alpha helical structure, 13 turns long, and three aromatic ring side chains of the are visible.

The specific amino acid composition of this pilin protein shown in FIG. 19 is the following 61 amino acid sequence take from the PDB entry:
PDB: 2M7G_A Structure of the Type IVa Major Pilin from the Electrically Conductive Bacterial Nanowires of *Geobacter sulfurreducens*:
ftliellivv aiigilaaia ipqfsavrvk aynsaassdl mlktalesa faddqtyppe s (SEQ ID NO: 12).

Xiao, et al., *supra*, indicates that aromatic rings provide the mechanism of conduction, and moreover, that the rings are arranged to be proximate in the 3-D structure to create a semi-continuous conducting path of aromatic rings. It is otherwise known in the field of conducting biological proteins that aromatic rings, often tyrosine, may play some role in conferring conductivity to proteins. Conductivity is at least hypothetically due to the presence of aromatic rings, which are electron rich and provide for high conduction around the rings.

For the peptides of the present disclosure, and based on observations of biological conductive proteins, aromatic amino acids can be added into the synthetic peptide sequences to enhance the conductivity of the synthetic peptide alpha helices. In various embodiments, tyrosine (Y) can be added. In certain aspects, the aromatic amino acids are added at locations that are proximate to each other in the 3-D alpha helical structure, to form a conduction path in which the aromatic rings are more closely and continuously spaced. One such example is shown in FIG. 18, where the alpha helix is based on the repeating motif EAYAR (SEQ ID NO: 3), thereby including tyrosine at positions i and i+5, which, as shown in FIG. 18, leading to a pattern of rings that spiral around the helix to provide a helical conduction enhancing path for electrons traversing the structure.

In various embodiments, and based on observations of biological conductive proteins, entire segments from an alpha helical biological conductive protein are incorporated into the synthetic alpha helical peptide as tandem repeats in order to enhance conductivity. These segments may be identical to the biological sequence, or highly similar to the biological sequence, such as at least 80%, at least 85%, at least 90%, at least 95%, or at least 98% sequence identity to the biological amino acid sequence. In various embodiments, such as in cases where the molecular wire will be used in an aqueous medium, such as in a biosensor, the portion of the sequence taken from or inspired by the biological protein should be a soluble or hydrophilic segment, or a segment that does not contain any of a transmembrane segment, in order to promote solubility of the helix and keep the molecular wire in solution in the molecular circuit, rather than displaced or precipitated onto the substrate, and to prevent precipitation or aggregation during handling in aqueous media, such as during self-assembly of the molecular wires into circuits, or assembly to other biomolecular components, such as attaching enzymes. In various embodiments outside the scope of the invention, a synthetic helical peptide molecular wire, based on the Pilin sequence PDB: 2M7G_A above, is formed based on extracting the 26 amino acid segments (underlined above), namely, QFSAYRVKAYNSAASSDLRNLKTALE (SEQ ID NO: 13), from the biological amino acid sequence and using this sequence as a repeating motif in the core of a synthetic peptide. This is a water-soluble domain, and a complete synthetic peptide having a molecular wire structure comprising 3 repeats of this domain, 1 instance of the sequence QFSAYRVKAYNSAASSDLRNLKTCLE (SEQ ID NO: 31), and the gold binding peptide sequence SEQ ID NO: 6, comprises the following amino acid sequence:

MHGKTQATSGTIQS-GSG-MHGKTQATSGTIQS-GSG-MHGKTQATSGTIQS-GSG-
QFSAYRVKAYNSAASSDLRNLKTALE-QFSAYRVKAYNSAASSDLRNLKTCLE-
QFSAYRVKAYNSAASSDLRNLKTALE-QFSAYRVKAYNSAASSDLRNLKTALE-GSG-
MHGKTQATSGTIQS-GSG-MHGKTQATSGTIQS-GSG-MHGKTQATSGTIQS (SEQ ID
NO: 14).

In various embodiments outside the scope of the invention, a synthetic peptide in accordance with the present disclosure comprises a sequence having at least 80%, at least 85%, at least 90%, at least 95%, or at least 98% sequence identity to SEQ ID NO: 14. In various embodiments outside the scope of the invention, the synthetic peptide comprises SEQ ID NO: 14. In various embodiments outside the scope of the invention, such a synthetic peptide functions as a conductive molecular wire for a molecular electronics circuit.

This construct comprising SEQ ID NO: 14 contains a helical segment based on 4 tandem repeats of the 26 amino acid biological segment, but wherein the second repeat includes a cysteine (C) replacing the A at position 24 of the motif. This internally located cystine acts as a specific conjugation site for a cysteine-maleimide conjugation reaction, such as to attach a binding probe to the synthetic peptide. Further, this helical segment of 104 amino acid is flanked on both N- and C-termini by triple tandem repeats of the gold binding peptide MHGKTQATSGTIQS (SEQ ID NO: 6), with flexible, soluble linkers -GSG- used to separate each repeat and the primary helix. In total, the length of the synthetic peptide having SEQ ID NO: 14 is 206 amino acids, the molecular weight is 21.37 kDa, the pI (protein isoelectric point, or pH of neutral charge) is 10.08, and the internal helical segment is 15.5 nm long, with approximately 28.9 helical turns.

In various embodiments, a synthetic peptide in accordance with the present disclosure comprises the following sequence of 187 amino acids:

QQSWPIS-GSG-QQSWPIS-GSG-ENLYFQG-GSG-QQSWPIS-GSG-A-EAAAR-EAAAR-
EAAAR-EAAAR-EAAAR-EAAAR-EAAAR-EAAAR-EAAAR-EAAAR-EACAR-
EAAAR-EAAAR-EAAAR-EAAAR-EAAAR-EAAAR-EAAAR-EAAAR-EAAAR-
EAAAR-A-GSG-QQSWPIS-GSG-ENLYFQG-GSG-QQSWPIS-GSG-QQSWPIS (SEQ ID
NO: 15).

This synthetic peptide is based on the helical motif EAAAR (SEQ ID NO: 1). It has an alpha-helical segment 15.6nm long, based on the amino acid lengths and the known alpha-helix pitch of 0.54 nm per turn, and 3.6 amino acids per turn. The sequence further features triple repeats of the palladium binding peptide QQSWPIS (SEQ ID NO: 16) on each end, separated by GSG linkers. The sequence EACAR (SEQ ID NO: 17) is a helical motif positioned at the center of the synthetic peptide. This helical motif is EAAAR (SEQ ID NO: 1) modified with a centrally located single cysteine in place of alanine (A) to provide a conjugation site in the synthetic peptide, such as for example, to use in maleimide or APN-based conjugation reactions. Although in SEQ ID NO: 15 the modified helical motif EACAR (SEQ ID NO: 17) is positioned precisely at the midpoint of the sequence, this example should not be seen as limiting, as the site specific conjugation site, in this case a C, can be shifted to either direction in the sequence. At positions 41 and 147, a single alanine (A) residue is present at each end of the primary alpha helix to avoid attachment of the primary alpha helix directly to the linker GSG, and possibly disruption of the secondary structure at the ends of the helix. Instead, the intervening alanine A provides a transitional helical-promoting amino acid, and in some ways serve as a sacrificial site in the helical structure. SEQ ID NO: 15 also includes a TEV protease cleavage sequence, ENLYFQG (SEQ ID NO: 18), added to provide the option to cleave off the multiple palladium binding peptide sequences. Other TEV protease cleavage sequences include replacement of the G at the P1' position with any one of S, A, M or C.

In various embodiments, a synthetic peptide in accordance with the present disclosure comprises a sequence having at least 85%, at least 90%, at least 95%, or at least 98% sequence identity to SEQ ID NO: 15. In various embodiments, the synthetic peptide comprises SEQ ID NO: 15. In various embodiments, such a synthetic peptide functions as a conductive molecular wire for a molecular electronics circuit.

In various embodiments, a synthetic peptide in accordance with the present disclosure comprises the following sequence of 164 amino acids:

QQSWPIS-GSG-QQSWPIS-GSG-QQSWPIS-GSG-EEEERRRR-EEEERRRR EEEERRRR-
EEEERRRR-EEEERRRR-EEEERRRR-EEEECRRR-EEEERRRR-EEEERRRR-
EEEERRRR-EEEERRRR-EEEERRRR-EEEERRRR-GSG-QQSWPIS-GSG-QQSWPIS-
GSG-QQSWPIS (SEQ ID NO: 19).

This synthetic peptide is based on the helical motif EEEERRRR (SEQ ID NO: 2). It has an alpha-helical segment 15.75nm long, based on the amino acid lengths and the known alpha-helix pitch of 0.54 nm per turn, and 3.6 amino acids per turn. The sequence further features triple repeats of the palladium binding peptide QQSWPIS (SEQ ID NO: 16) on each end, separated by GSG linkers. The sequence EEEECRRR (SEQ ID NO: 20) is a helical motif positioned at the center of the synthetic peptide. This helical motif is EEEERRRR (SEQ ID NO: 2) modified with an approximately centrally located single cysteine in place of arginine (R) to provide a conjugation site in the synthetic peptide, such as for example, to use in maleimide or APN-based conjugation reactions.

In various embodiments, a synthetic peptide in accordance with the present disclosure comprises a sequence having at least 85%, at least 90%, at least 95%, or at least 98% sequence identity to SEQ ID NO: 19. In various embodiments, the synthetic peptide comprises SEQ ID NO: 19. In various embodiments, such a synthetic peptide functions as a conductive molecular wire for a molecular electronics circuit.

In various embodiments outside the scope of the invention, a synthetic peptide comprises the following sequence of 227 amino acids:

QQSWPIS-GSG-QQSWPIS-GSG-QQSWPIS-GSG-A-EAAAR-EAAAR-EAAAR-EAAAR-
EAAAR-EAAAR-EAAAR-EAAAR-EAAAR-EAAAR-EAAAR-EAAAR-
EAAAR-EAAAR-EAAAR-EACAR-EAAAR-EAAAR-EAAAR-EAAAR-EAAAR-
EAAAR-EAAAR-EAAAR-EAAAR-EAAAR-EAAAR-EAAAR-EAAAR-
EAAAR-EAAAR-A-GSG-QQSWPIS-GSG-QQSWPIS-GSG-QQSWPIS (SEQ ID NO: 21).

This synthetic peptide is based on the helical motif EAAAR (SEQ ID NO: 1). It has an alpha-helical segment 25.4nm long, based on the amino acid lengths and the known alpha-helix pitch of 0.54 nm per turn, and 3.6 amino acids per turn. The sequence further features triple repeats of the palladium binding peptide QQSWPIS (SEQ ID NO: 16) on each end, separated by GSG linkers. The sequence EACAR (SEQ ID NO: 17) is a helical motif positioned at the center of the synthetic peptide. This helical motif is EAAAR (SEQ ID NO: 1) modified with a centrally located single cysteine in place of alanine (A) to provide a conjugation site in the synthetic peptide, such as for example, to use in maleimide or APN-based conjugation reactions. Although in SEQ ID NO: 21 the modified helical motif EACAR (SEQ ID NO: 17) is positioned precisely at the midpoint of the sequence, this example should not be seen as limiting, as the site specific conjugation site, in this case a C, can be shifted to either direction in the sequence. At positions 31 and 197, a single alanine (A) residue is present at each end of the primary alpha helix to avoid attachment of the primary alpha helix directly to the linker GSG, and possibly disruption of the secondary structure at the ends of the helix. Instead, the intervening alanine A provides a transitional helical-promoting amino acid, and in some ways serve as a sacrificial site in the helical structure.

In various embodiments outside the scope of the invention, a synthetic peptide comprises a sequence having at least 80%, at least 85%, at least 90%, at least 95%, or at least 98% sequence identify to SEQ ID NO: 21. In various embodiments outside the scope of the invention, the synthetic peptide comprises SEQ ID NO: 21. In various embodiments, such a synthetic peptide functions as a conductive molecular wire for a molecular electronics circuit.

It should be understood that other synthetic peptides capable of functioning as molecular wires are structurally conceivable using the same principles herein, and that the examples above are only to illustrate these principles and not to limit the scope. It is understood that there are other biological naturally occurring conductive protein helices besides Pilins on which to base such synthetic helical peptides, such as collagen filaments, dyneins, kinesins, components of molecular motors, elements of cytochromes, or the chains of immunoglobins.

### Structural variations in synthetic conductive peptides usable as molecular bridges in molecular electronic circuits

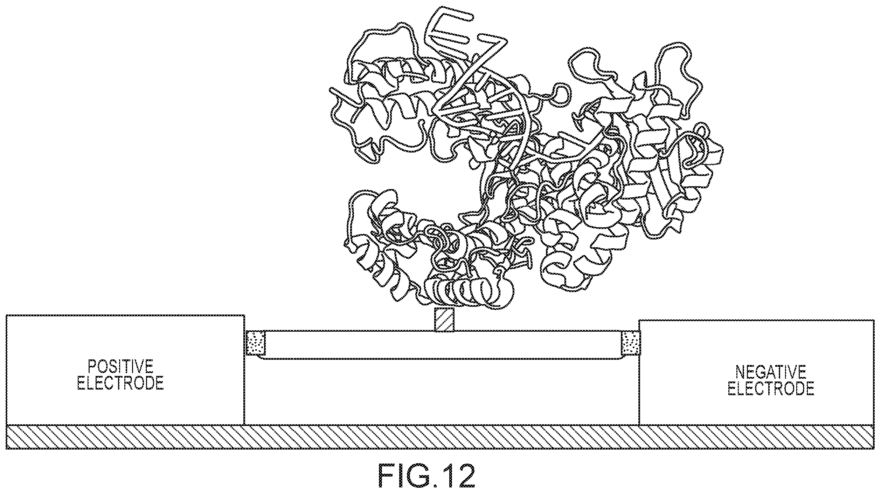

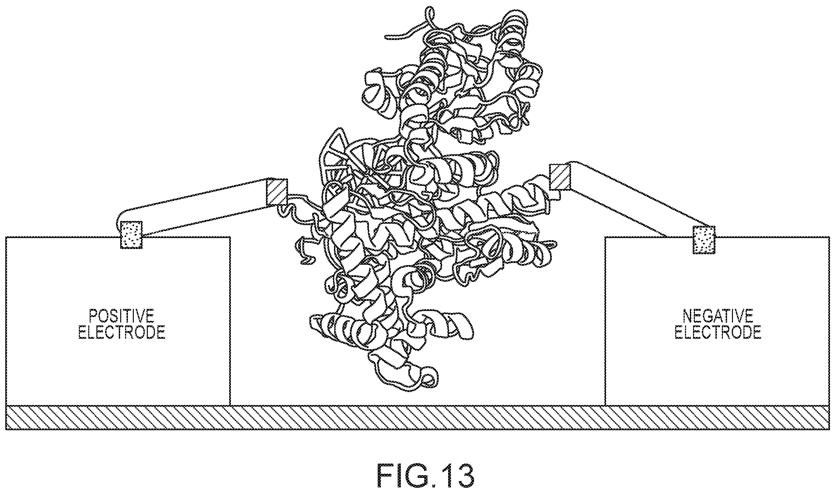

*Helical Bridge Length:* In various embodiments, a synthetic peptide herein can be designed to be a particular length by shortening or lengthening the one or more helical segments and/or by extending or reducing the repetition of the helical motifs. All helical motifs presented could be considered to define an arbitrarily long helix, for example the motif EAAAR (SEQ ID NO: 1) could be repeated without limit in a synthetic peptide, EAAAR....EAAAR, and any sequence segment taken from this could provide a bridge helix of any desired length. For peptide bridges that are used to span between spaced-apart electrodes, such as depicted in FIG. 1 or FIG. 12, the preferred lengths are from 5nm to 100nm, and more preferably from 10nm to 50nm, and most preferably from 15nm to 40nm. For synthetic peptides used as a connecting arm between a probe molecule and an electrode, such as depicted in FIG. 13, the preferred lengths are from 2nm to 50nm, and more preferably from 5nm to 30nm, and most preferably from 7nm to 20nm. For naturally occurring helical sequences, i.e. an alpha helix sequence occurring within a protein of biological origin, sub-segments taken from these may be used as shorter bridges, and sequences formed by repeating all or a sub-segment or segments of the helix sequence allow for longer bridges, relative to the naturally occurring alpha helix.

*Amino Acid Sequence Similarity:* For the helical amino acid sequences provided above, other preferred sequences that may have similar properties and utility include sequences having at least 80%, at least 85%, at least 90%, at least 95%, or at least 98% sequence identity to those sequences provided. Similarly, for any naturally occurring alpha helical sequence, a segment of at least 5 amino acids taken from this with at least 80%, at least 85%, at least 90%, at least 95%, or at least 98% sequence similarity to the natural form may provide a preferred bridge helix, or a segment that is repeated to create a bridge helix.

*Functionally Similar Amino Acid Substitutions:* For any of the helical sequences described herein, certain individual amino acid substitutions may be made that may be expected to retain the helical structure and other functionality. For example, replacement of one or more amino acids in the helix by amino acids that promote helical structure could be expected to provide a helix of similar structure and utility. It is known in particular that amino acids A, M, L, E and K promote helix formation, and in particular these could be substituted in for amino acids in the helix and likely retain a helical structure. More generally, scores have been developed for each amino acid, that rank their propensity to form helices or be compatible with helical structures, or to occur more frequently in naturally occurring helices. Substitutions of the similar or better scoring amino acids would be expected to preserve or even enhance helical structures. One such score is the Helical Propensity (reference: Pace, C, N., & Scholtz, J. M. (1998). A helix propensity scale based on experimental studies of peptides and proteins. Biophysical journal, 75(1), 422-427, which ranks the amino acids numerically from most helix compatible (low score) to least (high score) as: Ala = 0, Leu = 0.21, Arg = 0.21, Met = 0.24, Lys = 0.26, Gln = 0.39, Glu = 0.40, Ile = 0.41, Trp = 0.49, Ser = 0.50, Tyr = 0. 53, Phe = 0.54, Val = 0.61, His = 0.61, Asn = 0.65, Thr = 0.66, Cys = 0.68, Asp = 0.69, and Gly = 1.

In addition, salt bridges formed between oppositely charged amino acids located at i and i+4 or i+5 positions spatially adjacent in the helix, such as illustrated by the EAAAR (SEQ ID NO: 1) and EEEERRRR (SEQ ID NO: 2) motifs, could be formed by placement of any salt-bridging pair of amino acids, which generally include the positive charged ones, {R, K, H} salt-bridging to the negatively charged ones {E,D}. Thus, starting from any sequence as described herein, and substituting in one of {R, K, H} at position i in a sequence, and one of {E,D} at i+4 or i+5, would be expected to form a salt bridge, either maintaining one already present, or adding one for perhaps enhanced stability or conductivity. For example, the motif EAAAR (SEQ ID NO: 1) and EEEERRRR (SEQ ID NO: 2) described suggest functionally substituted motifs such as DAAAK (SEQ ID NO: 22), and EEDDRKHK (SEQ ID NO: 23). In cases of aromatic ring amino acids occurring in helixes, or in introducing such to potentially increase conductivity, any of the aromatic ring amino acids could be considered, such as F, W, V, or P.

More generally, classification of amino acids into similar groups, such as charged (positive, negative), polar, hydrophobic or hydrophilic, or aromatic, also guides a large number of substitutions replacing a given amino acid by one of a similar type, that would be expected to have similar functionality. All such substitutions are within the scope of modifications that could be applied to the exemplar sequences described herein.

*Nonstandard Amino Acid Substitutions.* Nonstandard amino acids (NAA), also known as unnatural amino acids (UAA), are amino acids other than the 22 that occur in biological proteins. In particular, there are many such NAA that are chemically modified forms of the standard amino acids, which could be used as alternatives to the standard amino acids. These can be incorporated into proteins using peptide chemical synthesis, of expression in expression systems that have implemented a nonstandard genetic code, or through chemical modifications made to a standard amino acid residing within a protein. Such NAA could still embody the important design principles of helical propensity, salt bridging, aromatic rings, spacer/linkers, or binding to electrodes---particularly when they are modified forms of the cognate standard ammo acids that embody these properties. Thus, substitutions for standard amino acids that are NAA of similar form provides a large class of alterations that could be made to the sequences described herein, which would be expected to provide similar structure and utility These NAA can be incorporated into proteins using peptide chemical synthesis, or by expression in expression systems that have implemented a nonstandard genetic code, or through chemical modifications made to a standard amino acid residing within a protein.

*Linker Variations:* In examples provided, the amino acid sequence GSG is used as a linker/spacer at the ends of the bridges, between the primary helix and between one or multiple binding groups at the ends. A great diversity of such linkers are known to those skilled in the art of protein engineering, where they are used to space out or reduce steric interference between functional domains of interest in a protein, and many of these could be used as alternative linkers/spacers in the sequences described herein. For example, other common linkers are G/S sequences such as G, GS, SG, GSGS (SEQ ID NO: 24), GSSSGSSSG (SEQ ID NO: 25), etc. More generally, {G,S} rich sequences provide convenient and commonly used linkers. More generally, a large diversity of linkers/spacers have been used in the literature.

In general, these linkers tend to be sequences that form flexible and hydrophilic chains, which can be conveniently done by short sequences of high {S,G} content. Preferably, such linkers are short, preferably, 1 to 10 amino acids, but longer ones may work as well. A compilation of various linkers/spacers used in diverse applications can be found at http://parts.igem.org/Protein_domains/Linker. In addition to peptide linkers/spacers such as GSG, other flexible molecular linkers could be used, in the case of peptides made by chemical synthesis, which allows for the addition of non-amino-acid elements into the peptide chain. One common such family would be short carbon chain linkers, such as C3, C6 or C12 (chains of 3, 6, or 12 hydrocarbons). These, or other short molecular linkers, could be used in place of peptide linkers such as GSG in such cases.

*Stapled Helices:* A known technique used to stabilize alpha-helices is the use of hydrocarbon staples between turns of the helix, to chemically link them together. Any of the helices described herein could also be modified to comprise one or multiple staples to further stabilize the helix. Such methods are described, for example, in: Hydrocarbon-Stapled Peptides: Principles, Practice, and Progress, Loren D. Walensky and Gregory H. Bird, Journal of Medicinal Chemistry, 57 (15), 6275-6288 (2014).

*Functionally Similar Motif Variations:* The helical motifs described herein have many direct extensions that embody the same design principles. For example, the motif EAAAR (SEQ ID NO: 1), would suggest functionally similar motifs EAAARRAAAE (SEQ ID NO: 26) or EAAARAARAAAR (SEQ ID NO: 27), etc., that use a helical forming amino acid, A, and have E-R salt-bridges that can form between i and i+4 locations. Or, for example, the motif EEEERRRR (SEQ ID NO: 2) would suggest as functionally similar motifs the longer form EEEERRRRRRRREEEE (SEQ ID NO: 28) which still pairs each E/R with an i+4 R/E. The motifs presented, EAAAR (SEQ ID NO: 1) and EEEERRRR (SEQ ID NO: 2) are just the simplest and/or shortest of these families, whereas many longer and/or more complex motif patterns would embody the same principles.

*General Bridge Architecture:* The scope of synthetic peptide structure beyond the specific example sequences provided herein, such as SEQ ID NOs: 15, and 19 , include synthetic peptides comprising the following general formula:
[X¹X²]ₘX³[X⁴]ₙX³[X²X¹]ₘ as defined in the claims,
wherein
each X¹ is independently a material binding peptide comprising 5 to 15 amino acids, a protease cleavage sequence, or a peptide capture tag;
each X² is independently a glycine/serine {G,S} rich linker or a C1-C20 carbon chain molecular linker;
each X³ is independently
   a metal binding group, or a material binding peptide comprising 5 to 15 amino acids;
each X⁴ is independently an alpha helix motif comprising 4 to 40 amino acids;
each m is independently 0 to 4; and
n is 1 to 40.

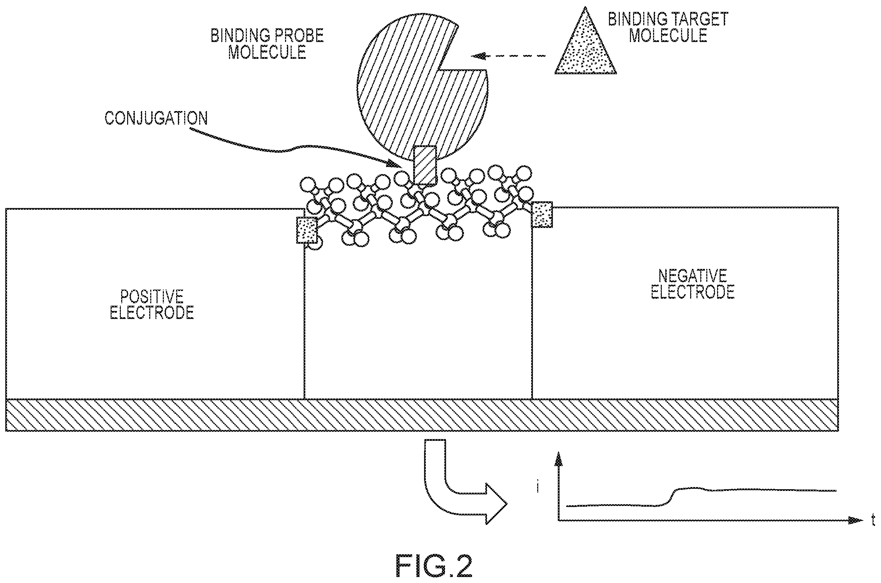

Each of the species of synthetic peptides encompassed within the genus [X¹X²]ₘX³[X⁴]ₙX³[X²X¹]ₘ, with each of the motifs and the scope of n and m defined by the claims, find use as a conductive molecular wires for bridging across spaced apart electrodes, such as illustrated in FIG. 2, or as synthetic peptide arm molecules that conjugate between an electrode and a specific site on a binding probe, such as illustrated in FIG. 13. The difference in the utility of the conducting synthetic peptide is dictated, at least in part, whether or not a conjugation site has been provided along the sequence of the synthetic peptide, such as for attaching a probe molecule to the synthetic peptide, and the configuration of the [X¹X²]ₘ and X³ motifs, such as whether these motifs are designed to bind to a metal electrode or to a biomolecule such as a binding probe and whether the termini of the synthetic peptide are identical or different. As discussed above, a synthetic bridge peptide is characterized in having a cysteine (C) or other conjugation site in the peptide sequence, such as near a midpoint along the length of the synthetic peptide. That is, at least one of the X⁴ alpha helical motifs may comprise a conjugation site, such as a cysteine residue. In various embodiments, the X¹ motif comprises a Material Binding Peptide, which might be arranged in triplicate with intervening glycine/serine-rich linkers X², such as -GSG-. In these cases when m is not 0, X³ may be used as a spacer between the triplicate arrangement of material binding sequences and the core alpha helical segment comprised of the repeating alpha helical motifs.

In other examples as discussed above, the entirety of one or both [X¹X²]ₘ terminal motifs may be absent altogether, *i.e.*, when m = 0. In various embodiments with m = 0, meaning the [X¹X²]ₘ segment is not present, X³ may take over the role of the binding motif in the terminus region of the synthetic peptide, either or both ends. For example, with m = 0, X³ may comprise a cysteine (so as to provide a single -SH group at the peptide terminus), or may comprise a more complicated arrangement for specific conjugation, such as a string of two or more cysteine amino acids, or a Material Binding Peptide. In other instances when m = 0, X³ may comprise an unnatural amino acid having an azide or other functional group for specific binding.

Although the general discussion above provides some perspective in how the motifs are chosen to impart various physical attributes to the synthetic peptide, the following options for the motifs further define the scope of the general formula of synthetic peptides, [X¹X²]ₘX³[X⁴]ₙX³[X²X¹]ₘ:

Each X¹ comprises any one of a material binding peptide comprising to 5 amino acids, a protease cleavage sequence, or a peptide capture tag. In certain examples, X¹ comprises an amino acid sequence having at least 80%, at least 85%, at least 90%, at least 95%, or at least 98% sequence identity to any one of SEQ ID NOs: 6, 7, 8, 9, 10, 11, 16, 18 or 29. In certain examples, X¹ comprises any one of SEQ ID NOs: 6, 7, 8, 9, 10, 11, 16, 18 or 29. In various embodiments, X¹ comprises a sequence configured for binding to a particular metal. It is important to remember than each instance of X¹ may be chosen independently so that the N- and the C- termini of the synthetic peptide can be different when necessary. X¹, when configured as a binding domain, could consist of material binding peptides, or a series of such material binding peptide sequences separated by intervening linkers X², or may comprise amino acids that bind/conjugate to certain materials or functional groups, such as cysteine that can bind to various metals or to the conjugation group maleimide. Binding domains could also comprise other known conjugation groups, such as thiol, biotin, maleimide, APN, lysine, azides, or amines, or many others known to those skilled in the art of conjugation chemistry. The peptide target tag could contain the peptide target of an antibody, such as a FLAG epitope tag, a HIS tag (poly-histidine), Myc tag, HA, GST, or other epitope tags, or peptide groups such as avitags, or aldehyde tags, that are conjugation targets. In other examples, X¹ may comprise a capture tag at one terminus of the peptide for synthesis reasons, or one or both termini may include a protease cleavage sequence such that the synthetic peptide can be digested away either prior to coupling to other elements, or after, such as from the electrodes or other circuit elements it was previously bonded to, or other biomolecules it may be conjugated to.

X² comprises a glycine/serine rich linker or a hydrocarbon-type linker. For the former, G, S, -GS-, and -GSG- are envisioned as non-limiting options, amongst other glycine/serine rich sequences, even up to at least 10 or more amino acids. The linker X² may also comprise "tethers," referred to as a "C1 to C20 carbon chain molecular linker." The only restriction to the nature of a C1 to C20 carbon chain molecular linker is that it be bivalent so that the linker can tether one portion of the synthetic peptide to another. In various embodiments, such linkers include, but are not limited to, methylene -CH₂-and its homologs, -(CH₂)*ₚ*-, wherein *p* = 1 to about 20, ethoxylates from 1 EO up to about 10 EO, 1,4-phenylene, -CO₂-, -C(O)-NH-, and so forth. The C1-C20 linkers may also include any combination of carbon atoms and heteroatoms, and may be acyclic, cyclic, aliphatic, or aromatic, or combinations thereof. In various embodiments, X² may comprise combinations of amino acid sequences and C1-C20 non-amino acid species. The length of X² may be customized for certain devices, such as depending on the contact area on a metal electrode, or to achieve a desired separation distance between a probe molecule bound to the synthetic peptide and metal electrodes. For example, X² may be purposely long in order to space a Material Binding Peptide X¹ far from a binding probe attached to about the midpoint of the synthetic peptide (*i.e.,* near the center of the [X⁴]ₙ segment).

In various embodiments, the motif X³ may comprise a metal binding group, or a material binding peptide comprising 5 to 15 amino acids. As discussed, the choice of X³ is at least partially dependent on the choices for X¹ and particularly if m = 0 and the terminus of the synthetic peptide is defined entirely by the nature of X³.

In various embodiments, X³ may comprise a single amino acid, such as an alanine (A), which functions as a spacer that would not compromise the alpha helical segment defined by [X⁴]ₙ. In various embodiments, X³ may comprise a longer spacer than just one amino acid and may comprise a transitional helical-promoting motif, a spacer that promotes alpha helical secondary conformation. Such a transitional helical-promoting motif may comprise up to about 5 amino acids, and may be as simple as a poly-alanine sequence.

In various embodiments, such as when m = 0, X³ may comprise a metal binding group. The choices for such a metal binding group have been discussed in detail herein, and include such species as a thiol group, a carbene, an amine group, a diazonium group, or any other functional group capable of binding at least to some degree to a metal such as Au, Pt or Pd. In various embodiments, the metal binding group may comprise an amino acid that provides a thiol group (i.e., cysteine), or an amino acid that is derivatized to include a functional group not native to the amino acid and capable of binding to a metal. Stated another way, X³ may comprise a single amino acid, but rather than the single amino acid chosen to function as a spacer that promotes alpha helical conformation, the single amino acid may be chosen because it provides a functional group capable of binding to metal. In various embodiments, X³ may comprise two or more cysteine residues, such as a string of six or more cysteines. In various embodiments, X³ may comprise a tetra-cysteine FLASH binding motif CCXXCC, (X = any amino acid), such as CCCGCC (SEQ ID NO: 5), as discussed above. In various embodiments, X³ may comprise a FLASH binding motif CCXXCC wherein the XX is proline-serine, namely CCPSCC (SEQ ID NO: 34).

In various embodiments, the helical core portion of the synthetic peptide, namely [X⁴]ₙ, may comprise repeats of any amino acid sequence that promote an alpha helical secondary structure to that portion, such as QFSAYRVKAYNSAASSDLRNLKTALE (SEQ ID NO: 13) or the repeating motifs comprising SEQ ID NOs: 1 or 2 as long as the conditions of claim 1 are fulfilled. As evident from the examples, the repeat integer n, and the sequence length of the repeated alpha helical motif X⁴, can have a profound effect on the overall length of the synthetic peptide, and both of these variables, along with the choices for the termini portions, can be manipulated to define very precise lengths for the synthetic peptide. As mentioned, a predictable and precise length for the synthetic peptide is important if the synthetic peptide is to be used as a molecule wire bridging across spaced-apart electrodes or connecting a biomolecule to an electrode in molecular electronic circuits.

The conjugation site within the helix, *e*.*g*., within at least one of the X⁴ alpha helical motifs that are repeated to define the alpha helical core of the synthetic peptide, could be an amino acid such as cysteine, or lysine, or it could be a modified amino acid, such as a lysine with a free azide group attached, or a lysine with a biotin attached, or a modified amino acid or non-amino acid site placed within the peptide by chemical modification or synthesis. In addition, and such primary conjugation sites may be chemically functionalized or converted to other groups for conjugation, such as a cysteine being converted to azide by use of an azide-maleimide bifunctional linker reacted to the cysteine. Many other such conversions from the primary conjugation site to a desired conjugation group are possible and well known to those skilled in the art of conjugation chemistry.

In various embodiments outside the scope of the invention, a synthetic peptide of the formula [X¹X²]ₘX³[X⁴]ₙX³[X²X¹]ₘ comprises an amino acid sequence with at least 80%, at least 85%, at least 90%, at least 95%, or at least 98% sequence identity to SEQ ID NO: 14. In a specific example, outside the scope of the invention, the synthetic peptide comprises SEQ ID No. 14.

In various embodiments, a synthetic peptide of the formula [X¹X²]ₘX³[X⁴]ₙX³[X²X¹]ₘ comprises an amino acid sequence with at least 85%, at least 90%, at least 95%, or at least 98% sequence identity to SEQ ID NO: 15. In a specific example, the synthetic peptide comprises SEQ ID NO. 15.

In various embodiments, a synthetic peptide of the formula [X¹X²]ₘX³[X⁴]ₙX³[X²X¹]ₘ comprises an amino acid sequence with at least 85%, at least 90%, at least 95%, or at least 98% sequence identity to SEQ ID NO: 19. In a specific example, the synthetic peptide comprises SEQ ID NO. 19.

In various embodiments outside the scope of the invention, a synthetic peptide of the formula [X¹X²]ₘX³[X⁴]ₙX³[X²X¹]ₘ comprises an amino acid sequence with at least 80%, at least 85%, at least 90%, at least 95%, or at least 98% sequence identity to SEQ ID NO: 21. In a specific example, outside the scope of the invention, the synthetic peptide comprises SEQ ID No. 21.

*Fabrication of Bridge Molecules:* Known methods of chemical peptide synthesis and protein expression can be used to produce the bridge molecules described herein. In the case of chemical peptide synthesis, where amino acids are added serially in chemical coupling steps to synthesize the chain of interest, it is possible to also add in modified/unnatural amino acids, as well as to add in internal or terminal non-amino acids groups, such as chemical linkers or conjugation groups. In the case of protein expression, the target bridge must be made entirely of amino acids, and a corresponding gene is produced, put into a biological expression system such as *E. coli* bacteria, and the gene is expressed to produce the resulting protein, which is then extracted and purified from the cultured cells. Such proteins can be further chemically modified to produce certain desired groups, such as reacting a cysteine with a maleimide to conjugate other groups at that site, or reacting of a lysine with NHS, or reacting with terminal amino or carboxy groups. Bridges made by either of these methods can generally be ordered from commercial vendors who perform these processes as services. Expression systems that have been genetically modified to use nonstandard amino acids can also be used to insert such NAA into the proteins products.

The principles herein also extend to molecular wires that are organized as bundles of single chain helical peptides described above. For example, a triple helix formed of three helical chains as outline above is a further extension of this concept. Such an example might be based on a synthetic form of collagen, which naturally forms a triple helix, for one such class of embodiments. Similarly, Pilins naturally organize into multi-chain superstructures, so similar multichain constructs may be formed from the synthetic molecular wires motivated by pilins.

### Use of synthetic peptides as molecular wires in molecular electronics

Conducting synthetic peptides in accordance with the present disclosure have at least two notable applications. They could be used as a molecular wire to form a conducting connection between other critical molecular circuit elements, such as is indicated in FIGS. 3, 4, 5 and 6. In these illustrated devices, the main function of the peptide is to provide a conductive path that does not overly impede the transport of electrons or other charge carriers through the circuit. In another notable application, the conductivity of the synthetic peptide varies due to interactions with its immediate environment, in which case it can act as part of a sensor construction.

In some embodiments, the wire interacts directly with molecules in the environment, such as gas molecules in a gaseous environment, or molecules in solution in a liquid environment, and changes conductivity or resistance as a result of these interactions (see FIG. 1). In other embodiments, such as shown in FIG. 2, a secondary molecule is conjugated to the principle molecular wire spanning the electrodes, and the interactions of this secondary molecule with its binding targets may create local environmental changes that modulate the conductivity of the wire, thereby providing a sensor for these interactions, as indicated in FIG. 2 by monitoring the current through this complex under an applied voltage. In such a sensor system, the local charge perturbations that result from the target substrate engaging with the enzyme perturb charge transport through the primary wire and are thus registered as changing in conductivity or current versus time, as indicated by the step-up change in the i vs. t current plot inset. Informative current changes could be an increase, or decrease, a pulse, or other time variations.

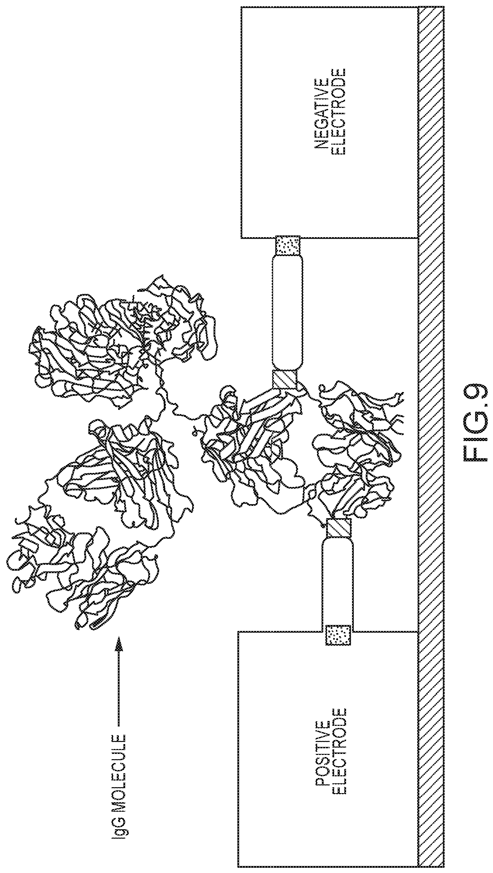

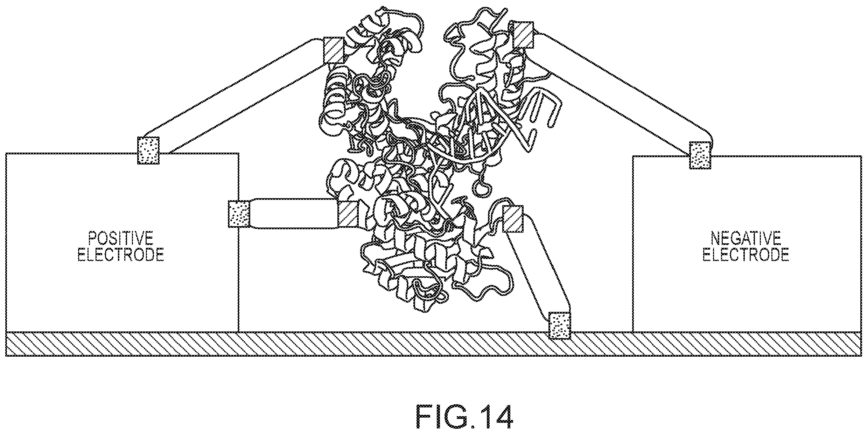

For purposes of illustration, and not limiting the scope of the present disclosure, FIGS. 2-10 and 12-14 illustrate a variety of such molecular sensor constructs comprising one or more of the synthetic peptides disclosed herein, acting as molecular wires in molecular electronic circuits, wherein the synthetic peptides are indicated schematically in the various figures as either ball-and-stick representations or (FIGS. 1-8) or as a long, thin bar (in FIGS. 9-10, and 12-14). Conjugation systems used to connect a synthetic peptide to an electrode and/or to other molecules are indicated schematically in the drawing figures by small shaded squares at the intersection of synthetic peptide/biomolecule and synthetic peptide/electrode or substrate. Such conjugation points may represent any known molecular conjugation for making specific connections between the two molecular/atomic (metal) components, such as click chemistry couplings, thiol-metal binding, biotin-avidin binding, material binding peptides, diazonium-metal binding, carbene-metal binding, cysteine-maleimide coupling, amine reactive crosslinking, or many others known to those skilled in the art of chemical conjugation, bioconjugation, or material surface conjugation. The cognate elements needed for this conjugation are typically integrated into the structure of the synthetic peptide as designed and manufactured herein and the attachment site. Briefly, electronic constructs comprising a synthetic peptide acting as a molecular wire are illustrated in the drawing figures as follows:
FIG. 2 shows a binding probe molecule conjugated to a synthetic peptide spanning two electrodes. This circuit acts as a sensor wherein, under an applied voltage, a current step-up signal (increase in conductivity) is produced by modulating the conductivity of the wire when the probe molecule binds to its cognate target molecule. In various embodiments, the binding probe molecule comprises an enzyme, such as a polymerase, and the sensor is usable to sequence DNA.

In various embodiments, the sensor of FIG. 2 comprises a processive enzyme molecular electronics sensor. In various examples the sensor comprises: a first electrode; a second electrode spaced apart from the first electrode by an electrode gap; a sensor complex comprising a processive enzyme conjugated to a synthetic peptide in accordance with the present disclosure, electrically connected to the first and second electrodes and bridging the gap there between. The processive enzyme may comprise a native or genetically engineered polymerase, reverse transcriptase, helicase, exonuclease, or molecular motor for packaging of viral DNA. For a working sensor, a trans-impedance amplifier is electrically connected to at least one of the first electrode and second electrode, the trans-impedance amplifier providing an output comprising a measurable electrical parameter; wherein the measurable electrical parameter comprises distinguishable signals corresponding to enzymatic activity of the processive enzyme.

FIG. 3 shows a configuration where the binding probe molecule is electrically connected to a pair of spaced-apart electrodes directly by two synthetic peptides acting as arm molecules, which here act primarily as conductive connectors to the probe molecule, which itself changes in conductivity when it binds its cognate target. Synthetic peptides herein for use as arm molecules only need to be functionalized at the termini since there is no need for conjugation from a central point along the sequence of the peptide. The configurations of either FIG. 2 or FIG. 3, incorporating synthetic peptides in accordance with the present disclosure, can be used as sensors for DNA sequencing.

In various embodiments, a molecular electronics circuit comprises first and second electrodes spaced apart by a nanogap on a substrate, and a synthetic peptide electrically connected to both first and second electrodes, thus bridging the nanogap. This embodiment is represented, for example, in FIG. 2. In various embodiments, the underlying substrate comprises SiO₂. In various embodiments, the electrodes comprise metals such as Au, Pt or Pd. In various embodiments, the first and second electrodes are positive and negative electrodes in the circuit. In various embodiments, the bridging synthetic peptide is conjugated to a binding probe, such as a polymerase enzyme. The conjugation site may be substantially centered on the synthetic peptide sequence so as to keep the attached polymerase from contacting either of the electrodes in the pair of electrodes. In various embodiments, a gate electrode may be positioned between the electrodes and below the bridging peptide, such as a buried gate electrode in the substrate. In various embodiments, a trans-impedance amplifier is electrically connected to at least one of the first electrode and second electrode, the trans-impedance amplifier providing an output comprising a measurable electrical parameter; wherein the measurable electrical parameter comprises distinguishable signals corresponding to binding events involving the binding probe conjugated to the bridging peptide.

In various embodiments, a molecular electronics circuit comprises first and second electrodes spaced apart by a nanogap on a substrate, and two synthetic peptides acting as arm molecules to electrically connect a binding probe, such as a polymerase enzyme, to both first and second electrodes, thus bridging the nanogap and forcing an electrically conductive pathway through a portion of the binding probe. This embodiment is represented, for example, in FIG. 3. In various embodiments, the underlying substrate comprises SiO₂. In various embodiments, the electrodes comprise metals such as Au, Pt or Pd. In various embodiments, the first and second electrodes are positive and negative electrodes in the circuit. The lengths of the two synthetic peptide arm molecules determines how the binding probe is centered between the electrodes, and these lengths may be engineered precisely in order to keep the attached polymerase from contacting either of the electrodes in the pair of electrodes. In various embodiments, a gate electrode may be positioned between the electrodes and below the bridging peptide, such as a buried gate electrode in the substrate. In various embodiments, a trans-impedance amplifier is electrically connected to at least one of the first electrode and second electrode, the trans-impedance amplifier providing an output comprising a measurable electrical parameter; wherein the measurable electrical parameter comprises distinguishable signals corresponding to binding events involving the binding probe electrically wired into the circuit by the peptide arm molecules.

Sensor circuits, such as these described in the context of FIGS. 2 and 3, and comprising one or more synthetic peptides in accordance with the present disclosure, may be arranged as arrays on chips, so that a plurality of such sensors can be operating as a system for DNA sequencing. In various aspects of a system, the system further comprises a CMOS sensor array chip comprising an array of the sensors and supporting pixel circuitry that performs measurement of the measurable electrical parameter.

In various aspects of a system, the system comprises at least two of the CMOS sensor array chips; an electronic hardware system for controlling and managing electrical inputs and data outputs of the chips; a fluidic system for introducing the synthetic DNA molecule in the buffer solution to the chips; and a signal processing and data recording system for capturing the distinguishable signals and for converting the distinguishable signals back to the information.

FIGS. 4-10 and 12-14 illustrate other configurations for molecular electronic circuits that comprise one or more synthetic peptides in accordance to various embodiments of the present disclosure. These circuits may be part of a biosensor as described above for FIGS. 2 and 3, and these sensors may be arranged in arrays on a chip as discussed.

FIG. 4 shows a special case of a molecular electronics circuit, wherein the probe molecule is a protein with an internal alpha helix as part of its structure, and with two synthetic peptides connecting ends of the internal alpha helix of the protein to positive and negative electrodes to provide a conducting pathway through a portion of the probe molecule.

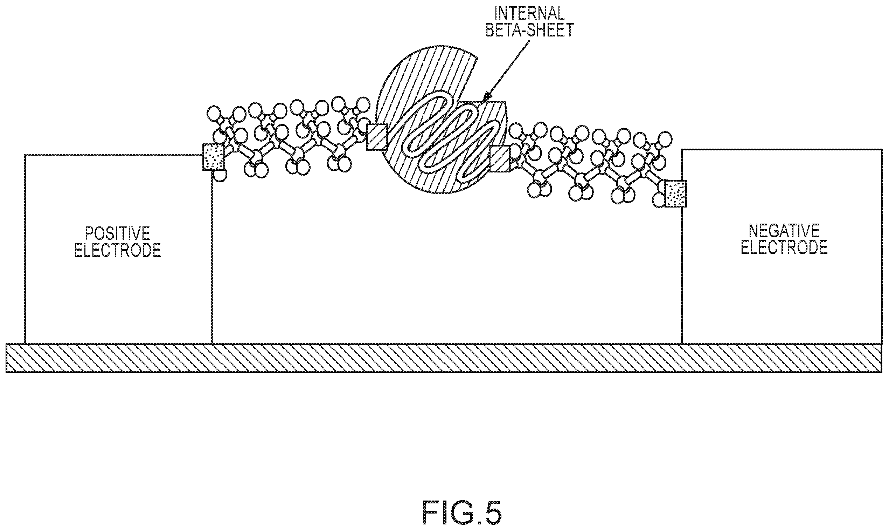

FIG. 5 shows a further variation, instead connecting the synthetic peptide arm molecules to an internal beta sheet in the protein probe molecule, again creating a preferred conduction path.

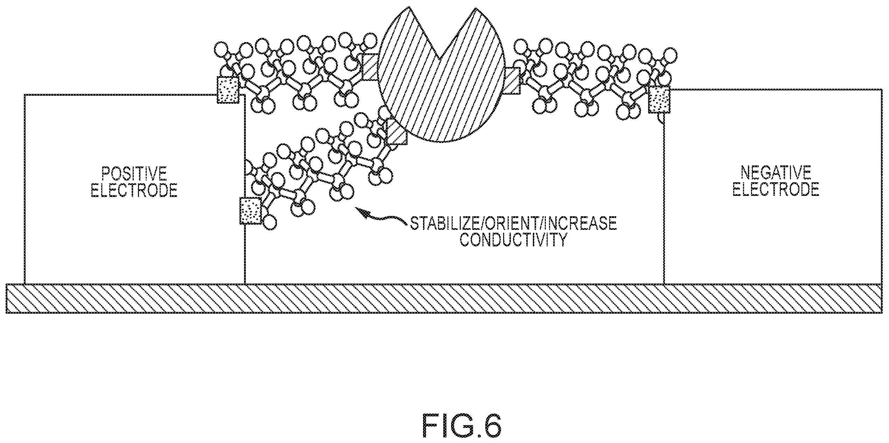

FIG. 6 shows a sensor configuration in which three synthetic peptide arm molecules are used to wire the probe to the electrodes with multiple connections to the electrodes.

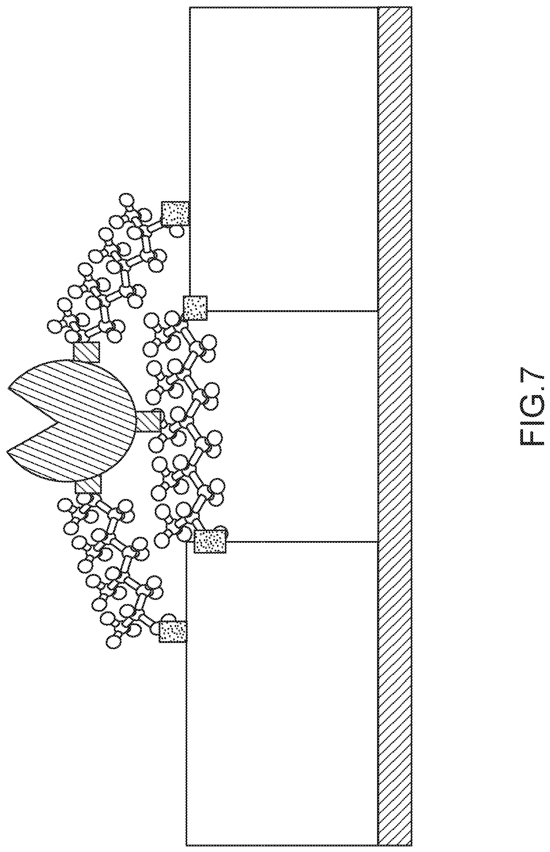

FIG. 7 shows the use of one synthetic peptide in the modulated fashion of FIG. 2, with two other synthetic peptides connecting the probe molecule directly to the first and second electrodes.

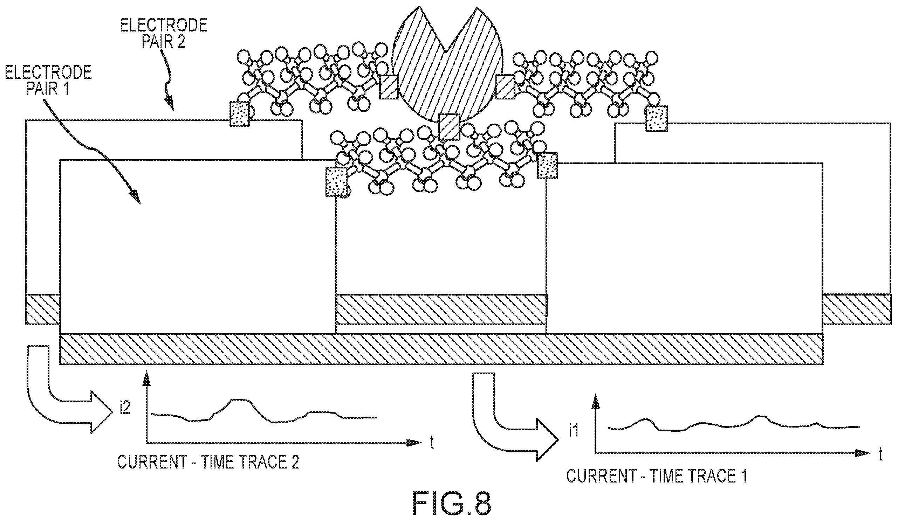

FIG. 8 shows a somewhat more complex configuration where the synthetic peptide arm molecules connect the probe to multiple electrode systems, allowing multiple simultaneous measurements.

FIG. 9 shows a specific case of FIG. 3, where the probe molecule is an IgG antibody protein, and the cognate binding target is the corresponding antigen, and where such a system can be used to detect antibody-antigen binding.

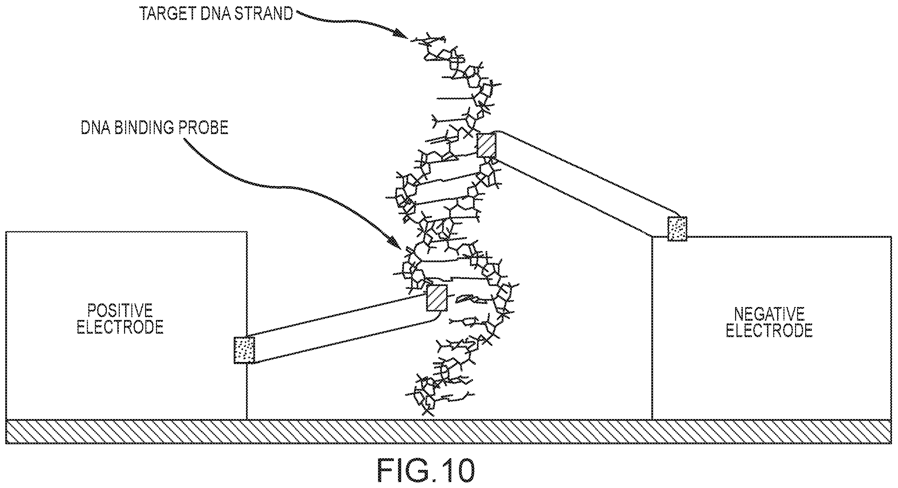

FIG. 10 shows a specific case of FIG. 3, where the probe molecule is a single stranded DNA oligonucleotide, and its binding target is the complementary oligo, and where such a system can be used to detect hybridization probe binding.

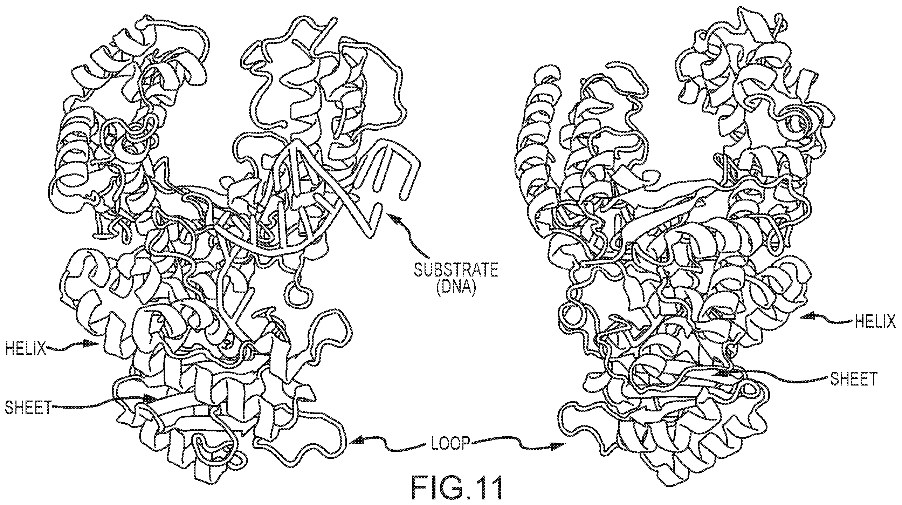

FIG. 11 shows the detailed protein structure of a Klenow Polymerase enzyme, which can be used as a binding probe in the context of sensing DNA sequence when the polymerase copies a template.

FIGS. 12, 13 and 14 show the use of synthetic peptides in accordance with the present disclosure to wire a polymerase enzyme in various configurations that are specific instance of FIGS. 2, 4 and 6, respectively, and which the resulting circuits could be used as sensors for the sequence of the template processes by the polymerase.

A conducting synthetic peptide used as a molecular wire in accordance with the present disclosure may be part of a molecular sensor complex used in a molecular electronics sensor. In any of the embodiments disclosed in FIGS. 2-14, bridging synthetic peptides and/or synthetic peptide arm molecules used in these circuits may comprise any one of the synthetic peptides disclosed herein. Molecular sensors that may utilize the synthetic peptides herein are also disclosed in U.S. Patent Nos. 9,829,456; 10,036,064; 10,125,420; 10,151,722 and 10,508,296; and U.S. Patent Application Publication No. 20180340220, Such sensors may be arranged in an array of sensors on a chip produced by CMOS processes. The sensors disclosed may be used for DNA sequencing.

In various embodiments, a method of sequencing a DNA molecule is disclosed. The method comprises: providing a circuit further comprising a positive electrode; a negative electrode spaced apart from the positive electrode; a conductive synthetic peptide electrically connected to the positive and negative electrodes; and a polymerase enzyme conjugated to the synthetic peptide at a conjugation site located along the synthetic peptide sequence; initiating at least one of a voltage or a current through the circuit; exposing the circuit to a solution containing primed single stranded DNA and/or dNTPs; and measuring electrical signals through the circuit as the polymerase engages and extends a template, wherein the electrical signals are processed to identify features that provide information on the underlying sequence of the DNA molecule processed by the polymerase. The synthetic peptide connected between the positive and negative electrode comprises the formula
[X¹X²]ₘX³[X⁴]ₙX³[X²X¹]ₘ as defined in the claims;
each X¹ is independently a material binding peptide comprising 5 to 15 amino acids, a protease cleavage sequence, or a peptide capture tag;
each X² is independently a glycine/serine {G,S} rich linker or a C1-C20 carbon chain molecular linker;
each X³ is independently
   a metal binding group, or a material binding peptide comprising about 5 to about 15 amino acids;
each X⁴ is independently an alpha helix motif comprising 4 to 40 amino acids;
each m is independently 0 to 4; and
n is 1 to 40,
wherein at least one instance of X⁴ comprises a conjugation site ;
   the conjugation site comprises a thiol, a biotin, an azide, an amine, a click chemistry group, cysteine, lysine or tyrosine.

In various embodiments, another method of sequencing a DNA molecule is disclosed. The method comprises: providing a circuit further comprising a positive electrode; a negative electrode spaced apart from the positive electrode; a first conductive synthetic peptide arm molecule electrically connected to the positive electrode and a first site on a polymerase enzyme and a second conductive synthetic peptide arm molecule electrically connected to the negative electrode and to a second site on the polymerase enzyme, thus providing a conductive pathway through the polymerase enzyme; initiating at least one of a voltage or a current through the circuit; exposing the circuit to a solution containing primed single stranded DNA and/or dNTPs; and measuring electrical signals through the circuit as the polymerase engages and extends a template, wherein the electrical signals are processed to identify features that provide information on the underlying sequence of the DNA molecule processed by the polymerase. In various embodiments, each one of the first and second synthetic peptide arm molecules comprise the formula:
[X¹X²]ₘX³[X⁴]ₙX³[X²X¹]ₘ as defined in the claims, wherein:
each X¹ is independently a material binding peptide comprising 5 to 15 amino acids, a protease cleavage sequence, or a peptide capture tag;
each X² is independently a glycine/serine {G,S} rich linker or a C1-C20 carbon chain molecular linker;
each X³ is independently,
   a metal binding group, or a material binding peptide comprising about 5 to about 15 amino acids;
each X⁴ is independently an alpha helix motif comprising 4 to 40 amino acids;
each m is independently 0 to 4; and
n is 1 to 40.

In various embodiments, at least one of [X¹X²]ₘ or X³ motifs of the synthetic peptide is configured to bind a terminus of the synthetic peptide to any one of the positive electrode, the negative electrode, the first site on the polymerase enzyme or the second site on the polymerase enzyme.

### EXAMPLES

### Experimental Validations of Bridge Utility:

Versions synthetic peptides comprising repeats the alpha helix motif EAAAR (SEQ ID NO: 1) and the alpha helix motif EEEERRRR (SEQ ID NO: 2) were fabricated, having palladium binding peptides at the termini, and a central cysteine residue usable for conjugation, and having lengths of approximately 15.6nm, 15.75nm and 25.4nm (for the alpha-helical portion of the bridge, not including linkers and binding peptides) and close to an integer number of helical turns, so that binding groups could contact electrode surfaces without generating excessive torsional stress on the helix. The three synthetic peptides used for these experiments were: 
QQSWPIS-GSG-QQSWPIS-GSG-ENLYFQG-GSG-QQSWPIS-GSG-A-EAAAR EAAAR-
EAAAR-EAAAR-EAAAR-EAAAR-EAAAR-EAAAR-EAAAR-EAAAR-EACAR-
EAAAR-EAAAR-EAAAR-EAAAR-EAAAR-EAAAR-EAAAR-EAAAR-EAAAR-
EAAAR-A-GSG-QQSWPIS-GSG-ENLYFQG-GSG-QQSWPIS-GSG-QQSWPIS (SEQ ID
NO: 15),
 
QQSWPIS-GSG-QQSWPIS-GSG-QQSWPIS-GSG-EEEERRRR-EEEERRRR EEEERRRR-
EEEERRRR-EEEERRRR-EEEERRRR-EEEECRRR-EEEERRRR-EEEERRRR-
EEEERRRR-EEEERRRR-EEEERRRR-EEEERRRR-GSG-QQSWPIS-GSG-QQSWPIS-
GSG-QQSWPIS (SEQ ID NO: 19),
 and

QQSWPIS-GSG-QQSWPIS-GSG-QQSWPIS-GSG-A-EAAAR-EAAAR-EAAAR-EAAAR-
EAAAR-EAAAR-EAAAR-EAAAR-EAAAR-EAAAR-EAAAR-EAAAR-
EAAAR-EAAAR-EAAAR-EACAR-EAAAR-EAAAR-EAAAR-EAAAR-EAAAR-
EAAAR-EAAAR-EAAAR-EAAAR-EAAAR-EAAAR-EAAAR-EAAAR-
EAAAR-EAAAR-A-GSG-QQSWPIS-GSG-QQSWPIS-GSG-QQSWPIS (SEQ ID NO: 21).

These synthetic peptides were fabricated using standard commercial protein expression services from Genscript, Inc. In brief, they were expressed in *E*. *coli.,* and included a FLAG tag added at the N-termini to allow for FLAG column purification. Materials were obtained purified to > 95%, and suspended in standard PBS buffer.

Furthermore, for purification purposes in producing these synthetic peptides by expression means, the FLAG epitope tag (the 7-mer peptide DYKDDDK (SEQ ID NO: 29)) was added to the N-terminal of this sequence, with a linker, as DYKDDDK-GSG- (SEQ ID NO: 30), so that a FLAG tag affinity column could be used to purify the expression products. This added epitope tag was left in place for the experimental work and therefore provides a site for anti-FLAG antibody binding, which can be used for anti-body based labelling purposes in experimental applications.

### Bridge Conductivity Experiments:

In the first experiment, a comparison of bridge conductivity between SEQ ID. NO: 15 and SEQ ID NO: 19 was performed. Nanoelectrodes were fabricated by e-beam lithography. In brief, resist was spin-coated onto a silicon wafer substrate, and e-beam lithography was used to expose the electrode pattern into the resist, defining a nanoelectrode pattern 50nm wide, with a 20nm tip-to-tip gap. The resist was developed, sputtering deposition was used to deposit a titanium adhesion layer 5nm thick and a palladium metal electrode layer 20nm thick. A lift-off process was used to produce the finished palladium nanoelectrodes.

Photolithography and lift-off methods were used to add palladium micro-electrodes that fan out to macroscopic pads for electrical connection, and additional photolithography was used to add a passivation layer of sputtered SiO₂, 100nm thick, leaving exposed only a 4-micron wide channel centered around the electrode gap, and exposing just 2 micron long portions of the nanowires for contact with solutions applied to the device.

The bridge peptides were put into solution onto these devices that were diced into small die (4mm x 8mm) containing pairs of 8 nanoelectrodes. A custom built flow cell and current measurement station was used to apply solutions to these devices, and measure electrode currents under a DC applied voltage. Devices were exposed to solutions containing 10nM concentration of bridge molecules, and allowed time for bridge binding to electrodes to occur. Currents through resulting electrodes were measured at an applied DC voltage of 1V. The difference between the current at 1V and 0V being defined as the "Delta Max" current.

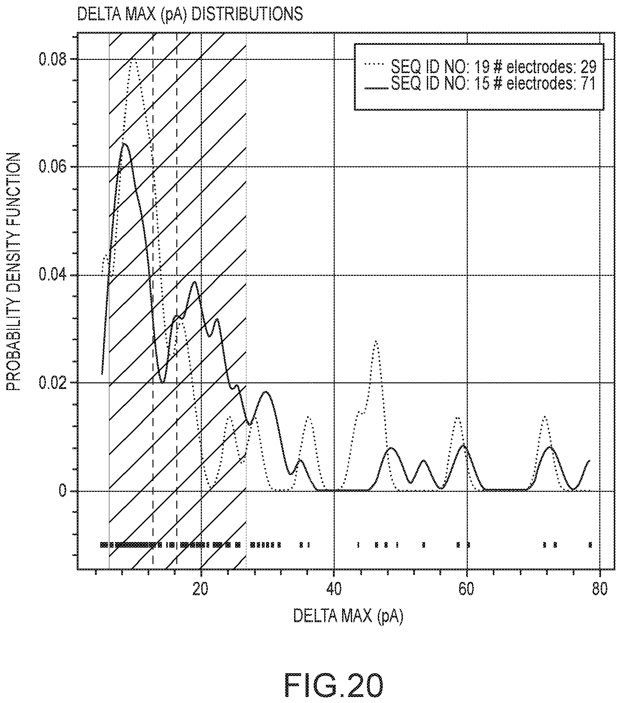

FIG. 20 shows the resulting current distributions of this Delta Max current, based on observations of 29 electrodes from SEQ ID NO: 19 bridges, and 71 electrodes from SEQ ID NO: 15 bridges. FIG. 20 shows the distribution of currents observed in pico-Amps (pA), and shows a higher conductivity for the SEQ ID NO: 15 peptide.

FIG. 20 shows that a first population of electrodes for SEQ ID NO: 19 and SEQ ID NO: 15 are centered below 4pA, and these are interpreted as probably not having formed a bridge spanning the electrodes. Other major populations of electrodes are seen to have current centered at 16pA (synthetic peptide having SEQ ID NO: 19) and 20pA (synthetic peptide having SEQ ID NO: 15) and these are interpreted as the conductivity observed in single bridges, and that SEQ ID NO: 15 therefore exhibits higher conductivity than SEQ ID NO: 19. Minor populations with higher order current, near 30pA and 45pA are interpreted as coming from multiple bridges spanning an electrode gap.

### Bridge Sensor Experiments:

In these experiments, the 25.4nm long reference synthetic peptide having SEQ ID NO: 21 was configured to create a sensor that detects binding and enzyme activity events. The central cysteine C in the synthetic peptide was conjugated to the 5' end of a linear single stranded DNA oligonucleotide using standard cysteine conjugation chemistry. The attached oligonucleotide acted as a probe molecule for binding to a primer DNA oligonucleotide. The resulting assembly of synthetic peptide and DNA oligonucleotide probe molecule was bridged across palladium electrodes, which were fabricated and deployed for experiments as in the bridge conductivity experiments described above. Once established in solution, this bridge/probe assembly was first allowed to bind to the complementary primer oligonucleotide, providing a first type of binding event to detect, and which established a 3' end priming site that is 5 bases removed from the conjugation junction site where the template oligonucleotide 5' end meets the cysteine of the bridging peptide. Then, polymerase was introduced to solution, allowing the polymerase to bind to the primer site, and providing a second type of binding event to detect. The polymerase was maintained in a non-catalytic buffer (with strontium used as divalent cation), so that it could not incorporate nucleotides, and they therefore bind and exit the pocket reversibly, providing yet a third type of binding event for the sensor to detect.

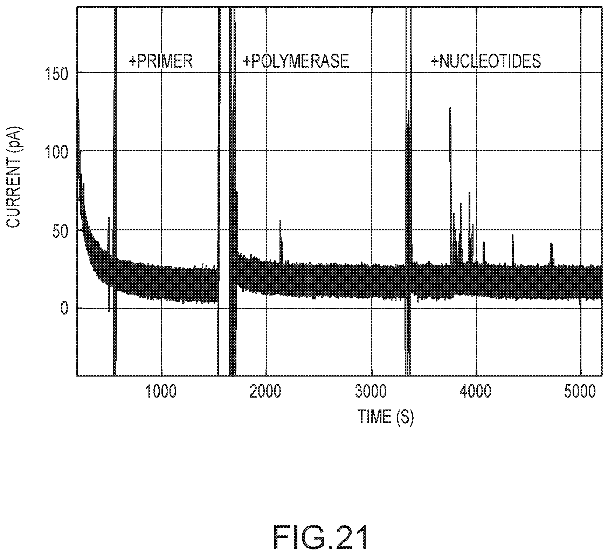

FIG. 21 shows current versus time trace of the entire experiment, which, over the course of 5000 seconds, shows that the sensor detects a strong current spike when the polymerase is added, and detects a series of strong spikes after the nucleotides are added. These spikes are interpreted as signaling polymerase binding to the primer complex on the bridge, and then of nucleotides non-productively and transiently binding into the polymerase pocket. This experiment illustrates the use of such a bridge peptide in a sensor context.

## Claims

1. A synthetic peptide comprising the formula:
[X¹X²]ₘX³[X⁴]ₙX³[X²X¹]ₘ
wherein
each X¹ independently comprises a material binding peptide comprising 5 to 15 amino acids, a protease cleavage sequence, or a peptide capture tag;
each X² independently comprises a glycine/serine {G,S} rich linker or a C1-C20 carbon chain molecular linker;
each X³ independently comprises a metal binding group, or a material binding peptide comprising 5 to 15 amino acids;
each X⁴ independently comprises an alpha helix motif comprising 4 to 40 amino acids;
each m is independently 0 to 4; and
n is 1 to 40;
wherein at least one instance of X¹ comprises an amino acid sequence having at least 85% sequence identity to SEQ ID NO: 16;
wherein at least one instance of X⁴ comprises a conjugation site and wherein the conjugation site comprises cysteine, lysine, tyrosine, a biotin, an azide, or a click chemistry group; and
wherein the synthetic peptide comprises an amino acid sequence with at least 85% sequence identity to SEQ ID NO: 15 or 19.

2. The synthetic peptide of claim 1, wherein at least one further instance of X¹ comprises an amino acid sequence having at least 80%, at least 85%, at least 90%, at least 95%, or at least 98% sequence identity to any one of SEQ ID NOs: 6, 7, 8, 9, 10, 11, 16, 18 or 29 and wherein at least one instance of X² comprises glycine, serine, GS, GSG, SEQ ID NO: 24, an amino acid sequence having at least 80% sequence identity to SEQ ID NO: 25, or a C1-C20 carbon chain molecular linker.

3. The synthetic peptide of claim 1, wherein in any one grouping of [X¹X²]ₘX³, if m ≠ 0, then X³ comprises a covalent bond, a single amino acid, or a transitional helical-promoting motif.

4. The synthetic peptide of claim 1, wherein the metal binding group comprises C, CC, CCC, SEQ ID NO: 32, SEQ ID NO: 33, SEQ ID NO: 5, SEQ ID NO: 35, or a FLASH binding motif of the sequence CCXXCC wherein X is any amino acid.

5. The synthetic peptide of claim 1, wherein at least one further instance of X⁴ comprises an amino acid sequence having at least 80%, at least 85%, at least 90%, at least 95%, or at least 98% sequence identity to any one of SEQ ID NOs: 1, 2, 3, 4, 13, 17, 20, 22, 23, 26, 27, 28 or 31 or an amino acid sequence with at least 80%, at least 85%, at least 90%, at least 95%, or at least 98% sequence identity to SEQ ID NO: 14 or an amino acid sequence with at least 80%, at least 85%, at least 90%, at least 95%, or at least 98% sequence identity to SEQ ID NO: 15.

6. The synthetic peptide of claim 1 comprising an amino acid sequence with at least 90%, at least 95%, or at least 98% sequence identity to SEQ ID NO: 19.

7. A molecular electronics circuit comprising:
a first electrode;
a second electrode spaced apart from the first electrode by a nanogap;
a bridging molecular wire comprising a synthetic peptide according to claim 1, electrically connected to both the first and second electrodes to bridge the nanogap; and
a polymerase enzyme conjugated to the conjugation site,
wherein the circuit includes a conductive pathway through the synthetic peptide.

8. A sensor comprising:
the molecular electronics circuit of claim 7; and
a trans-impedance amplifier is electrically connected to at least one of the first electrode and second electrode, the trans-impedance amplifier providing an output comprising a measurable electrical parameter.

9. A method of sequencing a DNA molecule, comprising:
providing the sensor of claim 8;
initiating at least one of a voltage or a current through the circuit;
exposing the circuit to a solution containing primed single stranded DNA and/or dNTPs; and
measuring electrical signals through the circuit as the polymerase engages and extends a template,
wherein the electrical signals are processed to identify features that provide information on the underlying sequence of the DNA molecule processed by the polymerase.

10. A molecular electronics circuit comprising:
a first electrode and a second electrode spaced apart by a nanogap;
a first synthetic peptide according to claim 1 electrically connected between the first electrode and a first site of a polymerase enzyme;
a second synthetic peptide according to claim 1 electrically connected between the second electrode and a second site of the polymerase enzyme,
wherein the circuit includes a conductive pathway through a portion of the polymerase enzyme.

11. A sensor comprising:
the molecular electronics circuit of claim 10; and
a trans-impedance amplifier is electrically connected to at least one of the first electrode and second electrode, the trans-impedance amplifier providing an output comprising a measurable electrical parameter.

12. A method of sequencing a DNA molecule, comprising:
providing the sensor of claim 11;
initiating at least one of a voltage or a current through the circuit;
exposing the circuit to a solution containing primed single stranded DNA and/or dNTPs; and
measuring electrical signals through the circuit as the polymerase engages and extends a template,
wherein the electrical signals are processed to identify features that provide information on the underlying sequence of the DNA molecule processed by the polymerase.

## Patentansprüche

1. Synthetisches Peptid, umfassend die folgende Formel:
[X¹X²]ₘX³[X⁴]ₙX³[X²X¹]ₘ
worin
X¹ jeweils unabhängig ein Material-bindendes Peptid, umfassend 5 bis 15 Aminosäuren, eine Proteasespaltungssequenz oder eine Peptideinfangmarkierung umfasst;
X² jeweils unabhängig einen Glycin/Serin-{G,S}-reichen Linker oder einen C1-20-Kohlenstoffketten-Moleküllinker umfasst;
X³ jeweils unabhängig eine Metall-bindende Gruppe oder ein Material-bindendes Peptid, umfassend 5 bis 15 Aminosäuren, umfasst;
X⁴ jeweils unabhängig ein alpha-Helixmotiv, umfassend 4 bis 40 Aminosäuren, umfasst;
m jeweils unabhängig 0 bis 4 ist; und
n = 1 bis 40 ist;
wobei zumindest eine Instanz von X¹ eine Aminosäuresequenz umfasst, die eine Sequenzidentität von zumindest 85 % mit SEQ ID NO: 16 aufweist;
wobei zumindest eine Instanz von X⁴ eine Konjugationsstelle umfasst und wobei die Konjugationsstelle Cystein, Lysin, Tyrosin, ein Biotin, ein Azid oder eine Click-Chemie-Gruppe umfasst; und
wobei das synthetische Peptid eine Aminosäuresequenz mit einer Sequenzidentität von zumindest 85 % mit SEQ ID NO: 15 oder 19 umfasst.

2. Synthetisches Peptid nach Anspruch 1, wobei zumindest eine weitere Instanz von X¹ eine Aminosäuresequenz mit einer Sequenzidentität von zumindest 80 %, zumindest 85 %, zumindest 90 %, zumindest 95 % oder zumindest 98 % mit einer beliebigen aus SEQ ID NO: 6, 7, 8, 9, 10, 11, 16, 18 oder 29 umfasst und wobei zumindest eine Instanz von X² Glycin, Serin, GS, GSG, SEQ ID NO: 24, eine Aminosäuresequenz mit einer Sequenzidentität von zumindest 80 % mit SEQ ID NO: 25 oder einen C1-20-Kohlenstoffketten-Moleküllinker umfasst.

3. Synthetisches Peptid nach Anspruch 1, wobei in einer beliebigen Gruppierung von [X¹X²]ₘX³, falls m ≠ 0 ist, X³ dann eine kovalente Bindung, eine einzelne Aminosäure oder ein vorübergehendes Helix-förderndes Motiv umfasst.

4. Synthetisches Peptid nach Anspruch 1, wobei die Metall-bindende Gruppe C, CC, CCC, SEQ ID NO: 32, SEQ ID NO: 33, SEQ ID NO: 5, SEQ ID NO: 35 oder ein FLASH-Bindungsmotiv der Sequenz CCXXCC umfasst, wobei X eine beliebige Aminosäure ist.

5. Synthetisches Peptid nach Anspruch 1, wobei zumindest eine weitere Instanz von X⁴ eine Aminosäuresequenz mit einer Sequenzidentität von zumindest 80 %, zumindest 85 %, zumindest 90 %, zumindest 95 % oder zumindest 98 % mit einer beliebigen aus den SEQ ID NO: 1, 2, 3, 4, 13, 17, 20, 22, 23, 26, 27, 28 oder 31 oder eine Aminosäuresequenz mit einer Sequenzidentität von zumindest 80 %, zumindest 85 %, zumindest 90 %, zumindest 95 % oder zumindest 98 % mit SEQ ID NO: 14 oder eine Aminosäuresequenz mit einer Sequenzidentität von zumindest 80 %, zumindest 85 %, zumindest 90 %, zumindest 95 % oder zumindest 98 % mit SEQ ID NO: 15 umfasst.

6. Synthetisches Peptid nach Anspruch 1, umfassend eine Aminosäuresequenz mit einer Sequenzidentität von zumindest 90 %, zumindest 95 % oder zumindest 98 % mit SEQ ID NO: 19.

7. Molekulare Elektronikschaltung, umfassend:
eine erste Elektrode;
eine zweite Elektrode, die durch einen Nanospalt von der ersten Elektrode beabstandet ist;
einen überbrückenden molekularen Draht, umfassend ein synthetisches Peptid nach Anspruch 1, der mit der ersten sowie der zweiten Elektrode elektrisch verbunden ist, um den Nanospalt zu überbrücken; und
ein Polymeraseenzym, das an der Konjugationsstelle konjugiert ist,
wobei die Schaltung einen leitfähigen Pfad durch das synthetische Peptid umfasst.

8. Sensor, umfassend:
eine molekulare Elektronikschaltung nach Anspruch 7; und
einen Transimpedanzverstärker, der elektrisch mit zumindest einer aus der ersten Elektrode und der zweiten Elektrode verbunden ist, wobei der Transimpedanzverstärker ein Ausgabesignal, umfassend einen messbaren elektrischen Parameter, bereitstellt.

9. Verfahren zum Sequenzieren eines DNA-Moleküls, umfassend:
Bereitstellen eines Sensors nach Anspruch 8;
Initiieren von zumindest einem aus einer Spannung oder einem Strom durch die Schaltung;
Aussetzen der Schaltung gegenüber einer Lösung, enthaltend eine geprimte einzelsträngige DNA und/oder dNTPs; und
Messen von elektrischen Signalen durch die Schaltung, wenn die Polymerase eingreift und eine Matrize erweitert,
wobei die elektrischen Signale verarbeitet werden, um Merkmale zu identifizieren, die Informationen zu der zugrundeliegenden Sequenz des DNA-Moleküls bereitstellen, das durch die Polymerase verarbeitet wird.

10. Molekulare Elektronikschaltung, umfassend:
eine erste Elektrode und eine zweite Elektrode, die durch einen Nanospalt beabstandet sind;
ein erstes synthetisches Peptid nach Anspruch 1, das elektrisch zwischen der ersten Elektrode und einer ersten Stelle eines Polymeraseenzyms eingebunden ist;
ein zweites synthetisches Peptid nach Anspruch 1, das elektrisch zwischen der zweiten Elektrode und einer zweiten Stelle des Polymeraseenzyms eingebunden ist,
wobei die Schaltung einen leitfähigen Pfad durch einen Teil des Polymeraseenzyms umfasst.

11. Sensor, umfassend:
eine molekulare Elektronikschaltung nach Anspruch 10; und
einen Transimpedanzverstärker, der elektrisch mit zumindest einer aus der ersten Elektrode und der zweiten Elektrode verbunden ist, wobei der Transimpedanzverstärker ein Ausgabesignal bereitstellt, das einen messbaren elektrischen Parameter umfasst.

12. Verfahren zum Sequenzieren eines DNA-Moleküls, umfassend:
Bereitstellen eines Sensors nach Anspruch 11;
Initiieren von zumindest einem aus einer Spannung oder einem Strom durch die Schaltung;
Aussetzen der Schaltung gegenüber einer Lösung, enthaltend eine geprimte einzelsträngige DNA und/oder dNTPs; und
Messen von elektrischen Signalen durch die Schaltung, wenn die Polymerase eingreift und eine Matrize erweitert,
wobei die elektrischen Signale verarbeitet werden, um Merkmale zu identifizieren, die Informationen zu der zugrundeliegenden Sequenz des DNA-Moleküls bereitstellen, das durch die Polymerase verarbeitet wird.

## Revendications

1. Peptide de synthétique comprenant la formule :
[X¹X²]ₘX³[X⁴]ₙX³[X²X¹]ₘ
où
chaque X¹ comprend indépendamment un peptide de liaison à un matériau comprenant 5 à 15 acides aminés, une séquence de clivage de protéase, ou une étiquette de capture peptidique ;
chaque X² comprend indépendamment un fragment de liaison riche en glycine/sérine {G,S} ou un fragment de liaison moléculaire à chaîne carbonée C1-C20 ;
chaque X³ comprend indépendamment un groupe de liaison à un métal, ou un peptide de liaison à un matériau comprenant 5 à 15 acides aminés ;
chaque X⁴ comprend indépendamment un motif d'hélice alpha comprenant 4 à 40 acides aminés ;
chaque m est indépendamment compris entre 0 et 4 ; et
n est compris entre 1 et 40 ;
dans lequel au moins une instance de X¹ comprend une séquence d'acides aminés ayant au moins 85 % d'identité de séquence à SEQ ID NO : 16 ;
dans lequel au moins une instance de X⁴ comprend un site de conjugaison et dans lequel le site de conjugaison comprend une cystéine, une lysine, une tyrosine, une biotine, un azide ou un groupe de chimie click ; et
dans lequel le peptide synthétique comprend une séquence d'acides aminés ayant au moins 85 % d'identité de séquence à SEQ ID NO : 15 ou 19.

2. Peptide synthétique selon la revendication 1, dans lequel au moins une autre instance de X¹ comprend une séquence d'acides aminés ayant au moins 80 %, au moins 85 %, au moins 90 %, au moins 95 % ou au moins 98 % d'identité de séquence à l'une quelconque des SEQ ID NO : 6, 7, 8, 9, 10, 11, 16, 18 ou 29 et dans lequel au moins une instance de X² comprend une glycine, une sérine, GS, GSG, SEQ ID NO : 24, une séquence d'acides aminés ayant au moins 80 % d'identité de séquence à SEQ ID NO : 25, ou un fragment de liaison moléculaire à chaîne carbonée C1-C20.

3. Peptide synthétique selon la revendication 1, dans lequel dans un quelconque groupement de [X¹X²]ₘX³, si m ≠ 0*,* alors X³ comprend une liaison covalente, un acide aminé unique ou un motif favorisant une conformation hélicoïdale de transition.

4. Peptide synthétique selon la revendication 1, dans lequel le groupe de liaison à un métal comprend C, CC, CCC, SEQ ID NO : 32, SEQ ID NO : 33, SEQ ID NO : 5, SEQ ID NO : 35, ou un motif de liaison FLASH de la séquence CCXXCC où X est un acide aminé quelconque.

5. Peptide synthétique selon la revendication 1, dans lequel au moins une autre instance de X⁴ comprend une séquence d'acides aminés ayant au moins 80 %, au moins 85 %, au moins 90 %, au moins 95 % ou au moins 98 % d'identité de séquence à l'une quelconque des SEQ ID NO : 1, 2, 3, 4, 13, 17, 20, 22, 23, 26, 27, 28 ou 31 ou une séquence d'acides aminés avec au moins 80 %, au moins 85 %, au moins 90 %, au moins 95 % ou au moins 98 % d'identité de séquence à SEQ ID NO : 14 ou une séquence d'acides aminés avec au moins 80 %, au moins 85 %, au moins 90 %, au moins 95 % ou au moins 98 % d'identité de séquence à SEQ ID NO : 15.

6. Peptide synthétique selon la revendication 1 comprenant une séquence d'acides aminés avec au moins 90 %, au moins 95 % ou au moins 98 % d'identité de séquence à SEQ ID NO : 19.

7. Circuit électronique moléculaire comprenant :
une première électrode ;
une seconde électrode espacée de la première électrode par un nano-espacement ;
un fil moléculaire de pontage comprenant un peptide synthétique selon la revendication 1, connecté électriquement à la fois à la première et à la seconde électrode pour combler le nano-espacement ; et
une enzyme polymérase conjuguée au site de conjugaison,
dans lequel le circuit comporte une voie conductrice à travers le peptide synthétique.

8. Capteur comprenant :
le circuit électronique moléculaire selon la revendication 7 ; et
un amplificateur de trans-impédance connecté électriquement à au moins l'une de la première électrode et de la seconde électrode, l'amplificateur de trans-impédance fournissant une sortie comprenant un paramètre électrique mesurable.

9. Procédé de séquençage d'une molécule d'ADN, comprenant les étapes consistant à :
fournir le capteur selon la revendication 8 ;
initier au moins l'un d'une tension ou d'un courant à travers le circuit ;
exposer le circuit à une solution contenant un ADN simple brin amorcé et/ou des dNTP ; et
mesurer des signaux électriques à travers le circuit lorsque la polymérase met en prise et étend une matrice,
dans lequel les signaux électriques sont traités pour identifier des caractéristiques qui fournissent des informations sur la séquence sous-jacente de la molécule d'ADN traitée par la polymérase.

10. Circuit électronique moléculaire comprenant :
une première électrode et une seconde électrode espacées par un nano-espacement ;
un premier peptide synthétique selon la revendication 1 connecté électriquement entre la première électrode et un premier site d'une enzyme polymérase ;
un second peptide synthétique selon la revendication 1 connecté électriquement entre la seconde électrode et un second site de l'enzyme polymérase,
dans lequel le circuit comporte une voie conductrice à travers une partie de l'enzyme polymérase.

11. Capteur comprenant :
le circuit électronique moléculaire selon la revendication 10 ; et
un amplificateur de trans-impédance connecté électriquement à au moins l'une de la première électrode et de la seconde électrode, l'amplificateur de trans-impédance fournissant une sortie comprenant un paramètre électrique mesurable.

12. Procédé de séquençage d'une molécule d'ADN, comprenant les étapes consistant à :
fournir le capteur selon la revendication 11 ;
initier au moins l'un d'une tension ou d'un courant à travers le circuit ;
exposer le circuit à une solution contenant un ADN simple brin amorcé et/ou des dNTP ; et
mesurer des signaux électriques à travers le circuit lorsque la polymérase met en prise et étend une matrice,
dans lequel les signaux électriques sont traités pour identifier des caractéristiques qui fournissent des informations sur la séquence sous-jacente de la molécule d'ADN traitée par la polymérase.
